# EUROPEAN PATENT APPLICATION

(11) **EP 2 752 935 A1**
(43) Date of publication of application: **09.07.2014**
(21) Application number: 12825038.8
(22) Date of filing: 24.08.2012
(51) Int. Cl.: H01M 14/00, C09B 23/00, H01L 31/04

(54) **DYE-SENSITIZED SOLAR CELL AND SENSITIZING DYE**

(30) Priority: 25.08.2011 JP 2011183404
(71) Applicant: National Institute for Materials Science, Tsukuba-shi, Ibaraki 305-0047 (JP)
(72) Inventor: Han Liyuan, Tsukuba-shi Ibaraki 305-0047 (JP); Islam Ashraful, Tsukuba-shi Ibaraki 305-0047 (JP)
(74) Representative: Calamita, Roberto
(86) International application number: PCT/JP2012/071505
(87) International publication number: WO 2013/027838

(57) **Abstract**

Provided is a dye-sensitized solar cell in which the energy conversion efficiency is increased by adsorbing two different kinds of dyes to the surface of titanium oxide that constitutes a semiconductor electrode. In a dye-sensitized solar cell including a conductive support, a porous semiconductor layer having sensitizing dyes adsorbed onto this conductive support, a carrier transport layer and a counter electrode, the energy conversion efficiency is increased by adsorbing a mixture of a ruthenium complex, and an organic dye having a different molecular size and exhibiting a high open circuit voltage as a photoelectric conversion characteristic obtainable by the dye alone, onto the porous semiconductor layer.

## Description

### Technical Field

The present invention relates to a dye-sensitized solar cell, and more particularly, to a dye-sensitized solar cell having high energy conversion efficiency, and a sensitizing dye.

### Background Art

I n recent years, from the viewpoint of the global environmental problems such as global warming, attention has been paid to solar cells that can convert solar light energy as a clean energy source replacing fossil fuels, to electric energy.

Among solar cells that can efficiently convert sunlight to electricity, solar cells which have been currently put to practical use include inorganic solar cells for residential use, such as single crystal silicon, polycrystalline silicon, amorphous silicon, cadmium telluride, and indium selenide. Disadvantages of these inorganic solar cells include that, for example, silicon systems of very high purity are required so that the purification processes are complicated, a large number of processes are needed, and thus the production cost is high.

In this regard, as a new type of solar cell, a dye-sensitized solar cell was unveiled in 1991 by Grätzel and his colleagues, and this is expected to be an inexpensive, highly efficient solar cell (Non Patent Literature 1). In a Grätzel cell, a porous semiconductor layer of titanium oxide or the like having a sensitizing dye adsorbed thereon, and a carrier transport layer are laminated between a transparent electrode and a counter electrode respectively formed on two sheets of light transmitting substrates such as glass plates. Sunlight enters through a light transmitting substrate, passes through the transparent electrode, reaches the semiconductor layer, and excites the sensitizing dye. Excited electrons thus generated migrate to the transparent electrode via the semiconductor layer, and return to the counter electrode via an external electric circuit. The excited electrons are transported by ions in a carrier transport layer in the cell, and are thereby returned to the dye that is in an oxidized state. When such electron movement starting from the generation of excited electrons of the dye by solar light is repeated, solar light energy is converted to electric energy. Regarding the sensitizing dye, a ruthenium-pyridine complex is used, and examples include N719 and N3 of bipyridine complexes, and black dye of a terpyridine complex. N719 has a wide absorption wavelength range of 300 nm to 730 nm, and black dye has a wide absorption wavelength range of 300 nm to 860 nm; however, in general, the molar absorptivity is low, and the energy conversion efficiency also remains at about 9%.

Generally, in order to increase the energy conversion efficiency of a dye-sensitized solar cell, there may be employed measures such as that the wavelength range of light that is absorbed by the sensitizing dye is extended, or the amount of adsorption of the sensitizing dye to the semiconductor surface is increased so that a wide range of wavelengths of sunlight including from ultraviolet radiation to infrared radiation can be utilized, and thereby a larger amount of light is absorbed. As an example of the former measure, there has been disclosed a technology of selecting two or more kinds of sensitizing dyes having different absorption peak wavelengths, or two or more kinds of sensitizing dyes exhibiting the maximum incident photon-to-electron conversion efficiencies at different absorption wavelength regions, and adsorbing those sensitizing dyes as a mixture onto a semiconductor surface, or adsorbing the sensitizing dyes by laminating different dyes in layers, to thereby extend the absorption wavelength range of the sensitizing dyes (Patent Literatures 1 to 4). As an example of the latter measure, it has been suggested to mix two kinds of dyes which are organic dyes of the same class having similar structures but having different molecular sizes, and to adsorb the dyes onto a semiconductor surface. In this case, the dye having a smaller size is adsorbed so as to fill the gaps made when the dye having a larger size was adsorbed. Due to the similar structures, a unique aggregate structure is formed between the molecules, and thus the dyes are compactly adsorbed onto the semiconductor surface. In another example, when two kinds of dyes having different absorption peak wavelengths and different molecular weights are mixed and adsorbed onto a semiconductor, similarly the dye having a smaller molecular weight is adsorbed in the gaps made when the dye having a larger molecular weight was adsorbed. As such, when the amount of adsorption of the dyes is increased, the area on the semiconductor surface that is not covered with the dyes is decreased, and it is believed that an effect of reducing a recombination in which electrons injected from the sensitizing dyes to the semiconductor flow to the carrier transport layer that is in contact with the semiconductor surface, can also be expected (Patent Literatures 4 and 5). However, the energy conversion efficiency of the conventional dye-sensitized solar cells employing the measures described above is about 7% to 9%, and this efficiency is not so different from the efficiency obtained when a ruthenium-pyridine complex is used alone as a dye-sensitizing dye, while an additive effect, or a synergistic effect, of the respective characteristics of the two kinds of dyes adsorbed as a mixture cannot be obtained. Recently, it has been reported that a dye-sensitized solar cell in which a black dye of a ruthenium-pyridine complex and organic dye D-131 are mixed and adsorbed, exhibits excellent energy conversion efficiency (11.0%) (Non Patent Literatures 2). However, this energy conversion efficiency could not surpass the officially recognized energy conversion efficiency (11.1%) of a dye-sensitized solar cell that used a black dye alone.

Patent Literature 1: JP 2000-268892 A
Patent Literature 2: JP 2003-249279 A
Patent Literature 3: JP 2006-107885 A
Patent Literature 4: JP 2006-278112 A
Patent Literature 5: JP 2009-212035 A

Non Patent Literature 1: B. O'Regan, M. Graetzel, Nature, 353, pp. 737-740 (1991).
Non Patent Literature 2: R. Y. Ogura, et al., Appl. Phys. Lett. 94, 073308 (2009).
Non Patent Literature 3: C. Kim, et al., J. Org. Chem. 73, pp. 7072-7079 (2008).
Non Patent Literature 4: C. Li, et al., ChemSusChem 1, pp. 615-618 (2008).
Non Patent Literature 5: K. R. Justin Thomas, et al., Chem. Mater. 20, pp. 1830-1840 (2008).
Non Patent Literature 6: D. P. Hagberg, et al., J. Am. Chem. Soc. 130, pp. 6259-6266 (2008).
Non Patent Literature 7: S. Ito, et al., Adv. Mater. 18, pp. 1202-1205 (2006); T. Horiuchi, et al., J. Am. Chem. Soc. 126, pp. 12218-12219 (2004).
Non Patent Literature 8: W. H. Liu, et al., Chem. Commun. pp. 5152-5154 (2008).
Non Patent Literature 9: S. Kim, et al., Tetrahedron 63, pp. 11436-11443 (2007).
Non Patent Literature 10: M. Gratzel, et al., Chem. Eur. J. 16, pp. 1193-1201 (2010).
Non Patent Literature 11: Z-S. Wang, et al., Chem. Mater. 20, pp. 3993-4003 (2008).
Non Patent Literature 12: Nam-Gyu Park, et al., Synthetic Metals. 159, pp 2571-77(2009).
Non Patent Literature 13: T. Dentani, et.al., New J. Chem., 33, pp. 93-101 (2009)

### Summary of Invention

### Technical Problem

In general, when two kinds of dyes are adsorbed as a mixture to a semiconductor, an interaction occurs between the dye molecules, and the dye molecules may be associated with each other. Thus, there are a number of occasions in which electrons excited by sunlight flow to the respective dyes to recombine with holes or the like before electrons reach the conduction band of the semiconductor, thereby the route of excited electrons to move to the semiconductor is disrupted, and thus the conversion efficiency is rather decreased. An object of the present invention is to provide a dye-sensitized solar cell having excellent energy conversion efficiency by selecting, when two kinds of dyes are adsorbed as a mixture onto a semiconductor, a combination of dyes that can make best use of the characteristics of the respective dyes.

### Solution to Problem

According to an aspect of the present invention, there is provided a dye-sensitized solar cell including the following items (a) to (e):
(a) a conductive support;
(b) a porous semiconductor layer;
(c) two kinds of sensitizing dyes that are adsorbed to the porous semiconductor layer and have different molecular sizes, and at least one of which has at least one alkyl side chain in the molecule;
(d) a carrier transport layer; and
(e) a counter electrode.

Furthermore, it is desirable if the photovoltaic characteristic, open circuit voltage exhibited by at least one of the two kinds of sensitizing dyes alone is 0.65 V or more.

According to another aspect of the present invention, there is provided a dyes-sensitized solar cell including the following items (a) to (e):
(a) a conductive support;
(b) a porous semiconductor layer;
(c) two kinds of sensitizing dyes that are adsorbed to the porous semiconductor layer and have different molecular sizes, and at least one of which has a photovoltaic characteristic, open circuit voltage of 0.65 V or more that is exhibited by the dye alone;
(d) a carrier transport layer; and
(e) a counter electrode.

Here, at least one of the two kinds of sensitizing dyes may have at least one alkyl side chain in the molecule.

Furthermore, the molecular weight of the sensitizing dye having a smaller molecular size between the two kinds of sensitizing dyes may be 400 or less.

Here, in all of the aspects described above, at least one of the two kinds of sensitizing dyes may have at least one alkyl side chain in the molecule.

Furthermore, the sensitizing dye having a smaller molecular size between the two kinds of sensitizing dyes may be an organic dye represented by the following formula: 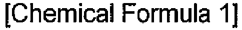 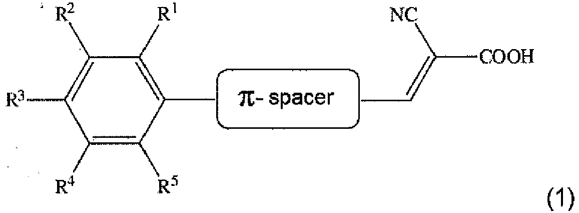 wherein R¹ to R⁵ each independently represent a hydrogen atom, an alkyl group having 1 to 18 carbon atoms, an alkoxy group, a dialkylamino group, or an alicyclic amino group; and the π-spacer represents a divalent phenylene group which may be substituted, or an aromatic heterocyclic group such as thiophene or thiazole.

Furthermore, at least one of R¹ to R⁵ may be an alkoxy group, a dialkylamino group or an alicyclic amino group, each having an alkyl side chain moiety having 3 to 16 carbon atoms.

Furthermore, the alkyl side chain moiety may be selected from the group consisting of a methyl group, an ethyl group, a propyl group, a butyl group, a pentyl group, a hexyl group, a heptyl group, an octyl group, a hexadecyl group, an isopropyl group, an isobutyl group, an isopentyl group, an isohexyl group, an isoheptyl group, an isooctyl group, a neopentyl group, a neohexyl group, a neoheptyl group, a neooctyl group, a sec-butyl group, a sec-pentyl group, a sec-hexyl group, a sec-heptyl group, a sec-octyl group, a tert-butyl group, a tert-pentyl group, a tert-hexyl group, a tert-heptyl group, a tert-octyl roup, a 2-ethylhexyl group, and a 1,1,3,3-tetramethylbutyl group.

Furthermore, the alicyclic amino group may be pyrrolidine or piperidine.

Furthermore, the aromatic heterocyclic group may have any one structure of the following formulas: 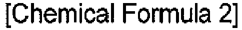 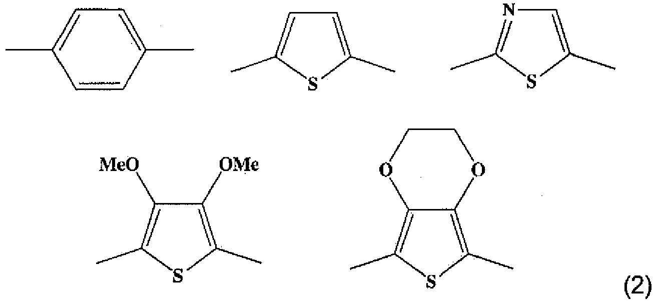 Furthermore, one of the two kinds of sensitizing dyes may be a ruthenium-pyridine complex.

Also, one of the two kinds of sensitizing dyes may be a terpyridine-based ruthenium complex.

According to another aspect of the present invention, there is provided a sensitizing dye represented by the above formula (1):

Here, at least one of R¹ to R⁵ may be an alkoxy group, a dialkylamino group or an alicyclic amino group, each having an alkyl side chain moiety having 3 to 16 carbon atoms.

Furthermore, the alkyl side chain moiety may be selected from the group consisting of a methyl group, an ethyl group, a propyl group, a butyl group, a pentyl group, a hexyl group, a heptyl group, an octyl group, a hexadecyl group, an isopropyl group, an isobutyl group, an isopentyl group, an isohexyl group, an isoheptyl group, an isooctyl group, a neopentyl group, a neohexyl group, a neoheptyl group, a neooctyl group, a sec-butyl group, a sec-pentyl group, a sec-hexyl group, a sec-heptyl group, a sec-octyl group, a tert-butyl group, a tert-pentyl group, a tert-hexyl group, a tert-heptyl group, a tert-octyl group, a 2-ethylhexyl group, and a 1,1,3,3-tetramethylbutyl group.

Furthermore, the alicyclic amino group may be pyrrolidine or piperidine.

Furthermore, the aromatic heterocyclic group may have any one structure of the above formula (2).

### Advantageous Effects of Invention

According to the present invention, when two kinds of dyes having different characteristics are adsorbed as a mixture onto a semiconductor, sunlight having a wide range of wavelengths can be absorbed, an interaction between the dye molecules is suppressed, and the density of the dyes adsorbing to the semiconductor surface is increased. Also, by increasing the energy gap between the LUMO and HOMO levels of the dyes and the conduction band of the semiconductor, excited electrons are caused to migrate efficiently to the conduction band of the semiconductor, and a recombination flowing from the conduction band to the carrier transport layer or to the LUMO and HOMO of the dyes is suppressed. As a result, the energy conversion efficiency of a dye-sensitized solar cell in which two kinds of dyes are adsorbed as a mixture to a porous semiconductor layer, can be increased.

### Brief Description of Drawings

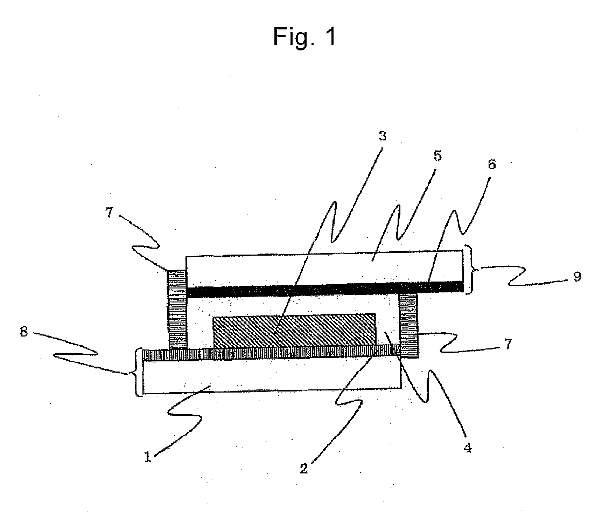

Fig. 1 is a schematic diagram illustrating the structure of the dye-sensitized solar cell of the present invention.

### Description of Embodiments

The inventors of the present invention intensively conducted studies to find a combination of dyes that can make best use of the respective characteristics when two kinds of dyes having different molecular sizes are adsorbed as a mixture to a porous semiconductor surface in a dye-sensitized solar cell. As a result, the inventors found that a combination of a ruthenium-pyridine complex and an organic dye that satisfies certain conditions brings about excellent energy conversion efficiency of a dye-sensitized solar cell.

On the transparent conductive side of a SnO₂ film-attached glass plate manufactured by Nippon Sheet Glass Co., Ltd., a commercially available titanium oxide paste (manufactured by Solaronix SA, Ti nanoxide T/SP) was applied by screen printing on the transparent conductive film to a film thickness of about 20 µm and an area of about 5 mm × 5 mm, and the paste was preliminarily dried for 30 minutes at 100°C, and then was calcined for 2 hours at 500°C in an air atmosphere. Thus, a titanium oxide film having a film thickness of 20 µm as a porous semiconductor layer was produced. A solution for adsorption was prepared by dissolving a dye to be screened and deoxycholic acid in ethanol at concentrations 2 × 10⁻⁴ M and 2 × 10⁻² M, respectively. The glass plate was immersed in this solution for 24 hours, and thereby the dye was adsorbed to the porous semiconductor layer. A counter electrode was produced by depositing 1 µm of a platinum film on the glass substrate described above, and the counter electrode and the porous semiconductor layer having the dye adsorbed thereto were arranged to face each other. The two members were tightly sealed, with a thermocompressed film spacer for preventing short circuit interposed therebetween. Thereafter, an acetonitrile solution of 1,2-dimethyl-3-propylimidazolium iodide (0.6 M), lithium iodide (0.1 M), iodine (0.05 M) and 4-tert-butylpyridine (0.5 M) as a liquid electrolyte was injected into the gap between the two electrodes to form a carrier transport layer, and thus a solar cell was produced. The solar cell thus obtained was irradiated with light (AM1.5, Solar Simulator) at an intensity of 100 mWcm⁻², and the current-voltage characteristics were measured. When a dye having a high open circuit voltage (for example, 0.65 V or more) is used in this dye-sensitized solar cell, the energy gap between the conduction band of the semiconductor and the LUMO level and the HOMO level of the dye becomes large, and therefore, a recombination in which electrons injected from the dye to the semiconductor return to the dye can be suppressed. Furthermore, in a dye-sensitized solar cell in which dyes having different molecular sizes are mixed and adsorbed together, the open circuit voltage is increased by the same effect, and the energy conversion efficiency is also expected to increase. A selected dye and a dye having a different molecular size were adsorbed as a mixture to a porous semiconductor, the photoelectric conversion characteristics were measured, and combinations that can make best use of the respective characteristics of the dyes were investigated. As a result, it was found that in order to obtain an additive effect of dyes that are adsorbed as a mixture with regard to the open circuit voltage of the photoelectric conversion characteristics, in a dye-sensitized solar cell in which two kinds of dyes having different molecular sizes are adsorbed as a mixture, it is desirable to have at least one of the dyes to contain at least one alkyl side chain. Alternatively, it was found that it is also desirable if the dye having a smaller molecular size has a molecular weight of 400 or less. Furthermore, it was also found that it is desirable if the open circuit voltage exhibited by each of the dyes when used alone is 0.6 V or more, and particularly preferably 0.65 V or more.

One dye of the two kinds of sensitizing dyes having different molecular sizes (hereinafter, referred to as sensitizing dye I) is preferably a ruthenium-pyridine complex, and more preferably N3 (formula (3)) or N719 (formula (4)) of a bipyridine complex, or a black dye (formula (5)) of a terpyridine complex. 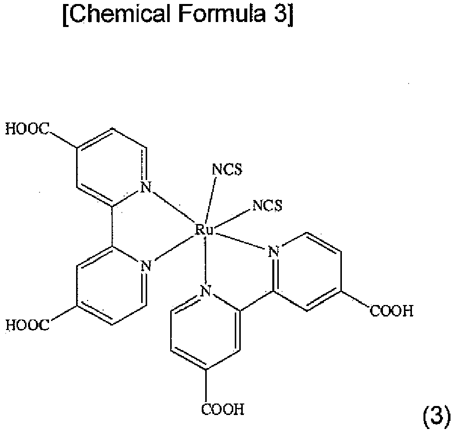 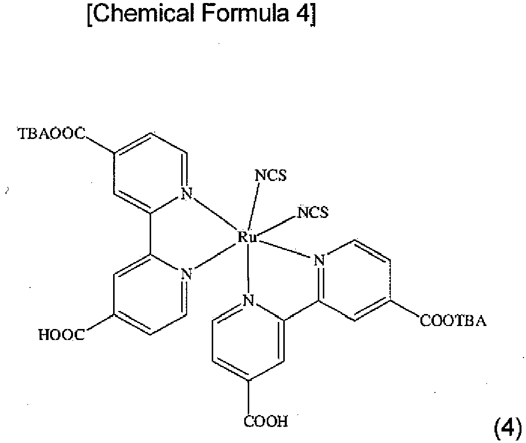 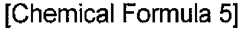 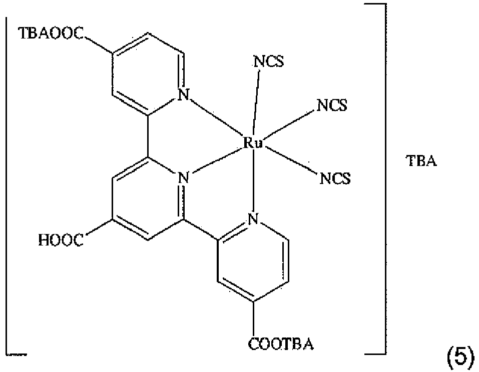 Furthermore, examples of the organic dye other than a ruthenium complex (hereinafter, referred to as sensitizing dye II), which exhibits an open circuit voltage of 0.65 V or more, between the two kinds of dyes having different molecular sizes, include a compound of (formula (6)) (Non Patent Literature 3), a compound of (formula (7)) (Non Patent Literature 4), a compound of (formula (8)) (Non Patent Literature 5), a compound of (formula (9)) (Non Patent Literature 6), a compound of (formula (10)) (Non Patent Literature 7), a compound of (formula (11)) (Non Patent Literature 8), a compound of (formula (12)) (Non Patent Literature 9), a compound of (formula (13)) (Non Patent Literature 10), a compound of (formula (14)) (Non Patent Literature 11), and a compound of formula (15) (Non Patent Literature 13) shown below. 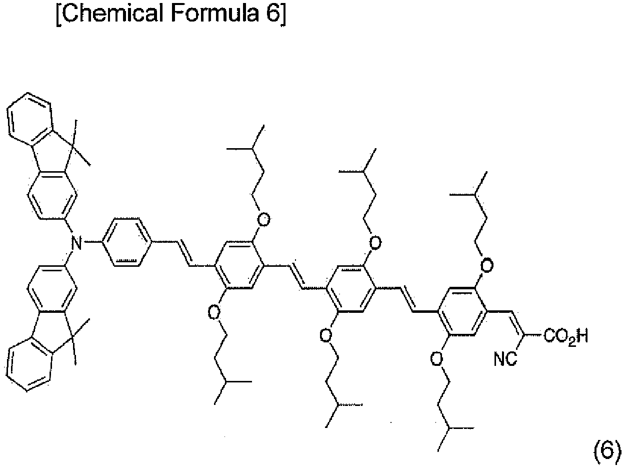 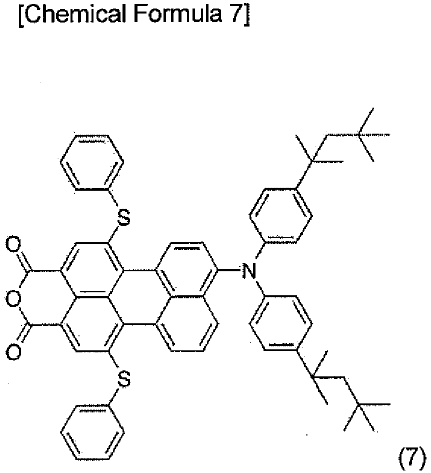 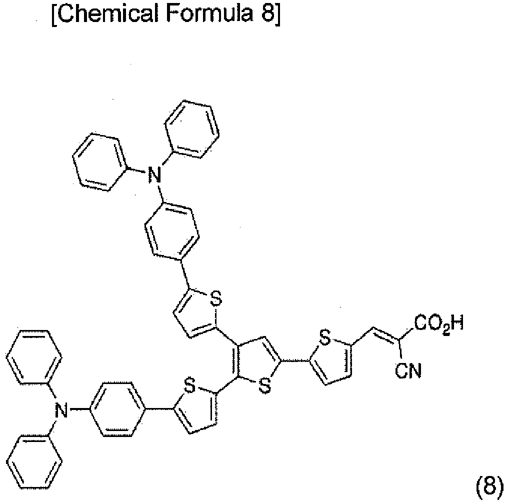 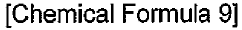 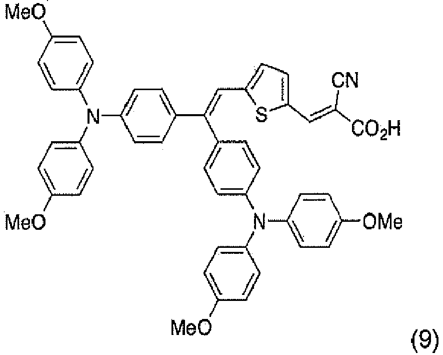 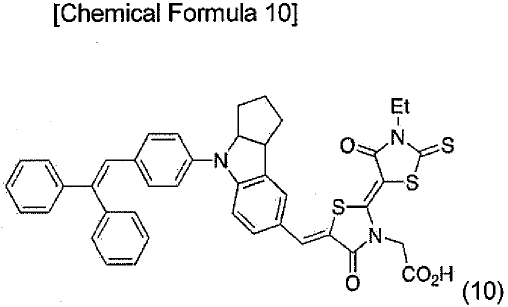 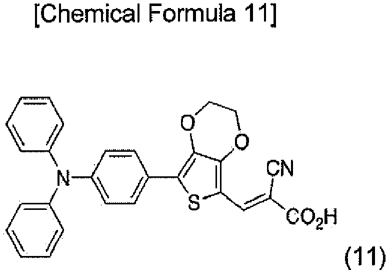 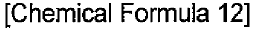 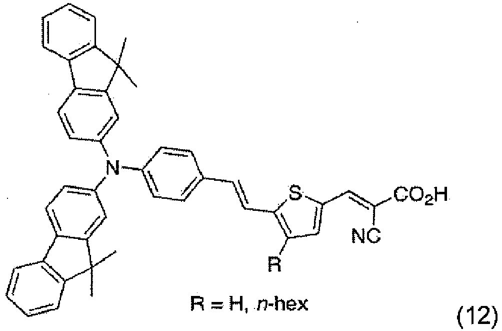 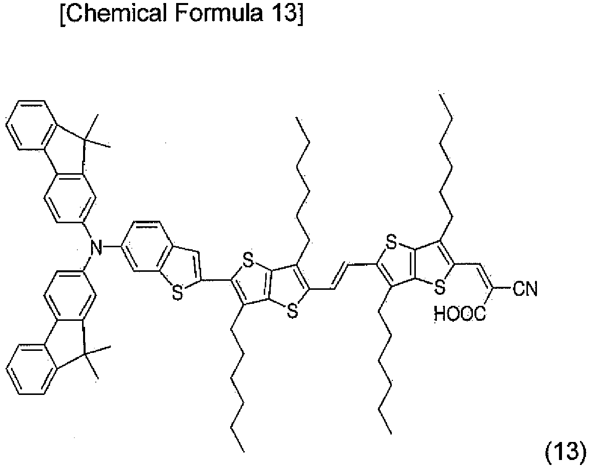 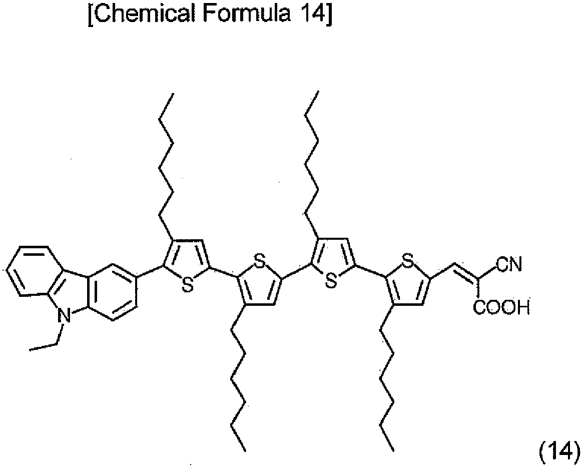 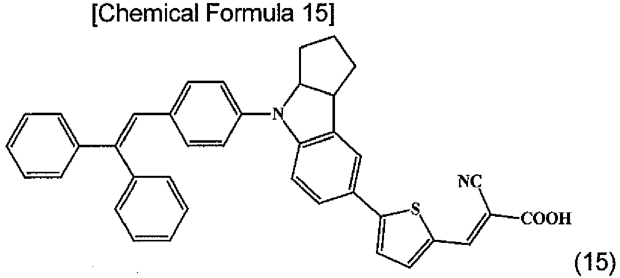 In regard to the organic dye (sensitizing dye II) that has at least one alkyl side chain in the molecule, the alkyl side chain is bonded to the parent nucleus of the dye through a linear or branched alkyl group (for example, the compounds of (formula (7)), (formula (12)) and (formula (13))), an alkoxy group (for example, the compounds of (formula (6)) and (formula (9))), a monoalkylamino group, or a dialkylamino group, and the alkyl chain may have therein an ether bond, a thioether bond, or an amino bond. The alkyl side chain has 1 to 18 carbon atoms, preferably 3 to 16 carbon atoms, and particularly preferably 4 to 8 carbon atoms. Specific examples of the alkyl side chain include alkyl groups such as a methyl group, an ethyl group, a propyl group, a butyl group, a pentyl group, a hexyl group, a heptyl group, an octyl group, a hexadecyl group, an isopropyl group, an isobutyl group, an isopentyl group, an isohexyl group, an isoheptyl group, an isooctyl group, a neopentyl group, a neohexyl group, a neoheptyl group, a neooctyl group, a sec-butyl group, a sec-pentyl group, a sec-hexyl group, a sec-heptyl group, a sec-octyl group, a tert-butyl group a tert-pentyl roup, a tert-hexyl group, a tert-heptyl group, a tert-octyl group, a 2-ethylhexyl group, and a 1,1,3,3-tetramethylbutyl group; and an alkoxy group, a monoalkylamino group and a dialkylamino group, all having the aforementioned alkyl groups.

Such an alkyl side chain prevents association between the dyes, and since a hydrophobic layer is formed between the semiconductor surface and the carrier transport layer, making it difficult for the electrolyte in the carrier transport layer to infiltrate, a recombination that flows between the semiconductor and the carrier transport layer can be suppressed.

Furthermore, as the dye having a smaller molecular size, an organic dye which has an open circuit voltage of 0.65 V or more, has an alkyl side chain in the molecule, and may have a molecular weight of 400 or less may be used, and an example thereof may be an organic dye represented by general formula (formula (16)): 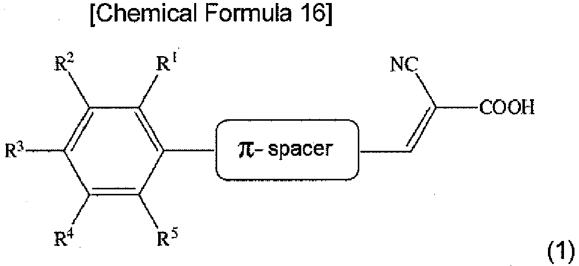 This sensitizing dye II is a [donor site-(π-spacer)-acceptor site] type organic dye represented by general formula (formula (16)), which employs 2-cyanoacrylic acid having a carboxyl group as an anchor group and a nitrile group as an auxiliary group in the molecule as an acceptor site in order to have the dye strongly adsorbed to the semiconductor surface, and in the formula, it is preferable that R¹ to R⁵ each independently represent a hydrogen atom, an alkyl group having 1 to 18 carbon atoms, an alkoxy group, a dialkylamino group, or an alicyclic amino group, while the number of carbon atoms in the alkyl side chain moiety of each of the functional groups is preferably 4 to 16. Examples include alkyl groups such as a methyl group, an ethyl group, a propyl group, a butyl group, a pentyl group, a hexyl group, a heptyl group, an octyl group, a hexadecyl group, an isopropyl group, an isobutyl group, an isopentyl group, an isohexyl group, an isoheptyl group, an isooctyl group, a neopentyl group, a neohexyl group, a neoheptyl group, a neooctyl group, a sec-butyl group, a sec-pentyl group, a sec-hexyl group, a sec-heptyl group, a sec-octyl group, a tert-butyl group, a tert-pentyl group, a tert-hexyl group, a tert-heptyl group, a tert-octyl group, a 2-ethylhexyl group, and a 1,1,3,3-tetramethylbutyl group. Furthermore, examples of the alicyclic amino group include pyrrolidine and piperidine. Also, the functional group is preferably an alkoxy group or a dialkylamino group.

The π-spacer moiety represents a divalent phenylene group which may be substituted, or an aromatic heterocyclic group such as thiophene or thiazole. Specific examples thereof include the following. 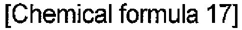 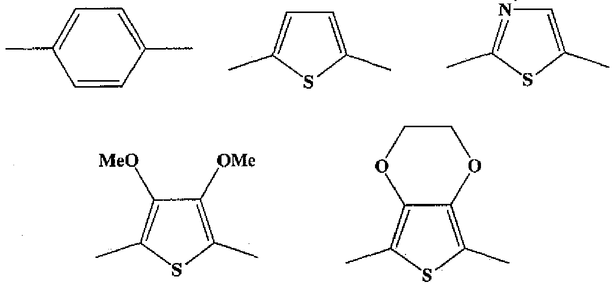 The sensitizing dye II represented by the general formula (formula (16)) described above may have a molecular weight of 400 or less, and the molecular size thereof can be reduced to the extent that the sensitizing dye II can be adsorbed in a manner of being embedded in the gaps formed as a dye having a larger molecular size (that is, sensitizing dye I) is adsorbed to a semiconductor surface. Furthermore, the LUMO of the dye lies in the acrylic acid part in proximity to the carboxyl group, and the HOMO lies in the terminal phenyl group part. Therefore, the electron distribution in an excited state is such that the electron density becomes maximal in the proximity of the carboxyl group, and excited electrons are efficiently injected into the semiconductor. Furthermore, in a dye having a high open circuit voltage, the energy gap between the LUMO level and HOMO level and the conduction band level of the semiconductor becomes larger, and a recombination in which the excited electrons injected from the dye to the semiconductor return to the LUMO or HOMO of the dye, can be prevented. Furthermore, when two kinds of dyes having different molecular sizes are adsorbed to the semiconductor, the dyes are adsorbed in a manner such that the dye having a smaller size is embedded in the gaps formed by the dye with a larger size being adsorbed. Therefore, the area that is not coated with dyes on the semiconductor surface is reduced, and a recombination in which excited electrons injected from the dyes flow from the semiconductor surface to the carrier transport layer, is suppressed. Furthermore, when a hydrophobic alkyl side chain is bonded onto a terminal phenyl group distal to the carboxyl group in the molecule, a hydrophobic layer caused by alkyl side chains is formed between the semiconductor surface and the carrier transport layer, it becomes difficult for the water-soluble electrolyte to infiltrate into the semiconductor surface, and thus a recombination is suppressed. It was confirmed that there is no interaction between these selected two kinds of dyes, since absorption peaks of the adsorbed dyes are respectively observed alone in the UV-VIS spectrum of a semiconductor having the two kinds of dyes adsorbed as a mixture. As a result, a dye-sensitized solar cell having a semiconductor electrode on which selected two kinds of dyes are adsorbed as a mixture, could exhibit excellent energy conversion efficiency due to a synergistic effect of the respective dyes.

There are no particular limitations on the method of synthesizing the dye represented by general formula (formula (16)) of the present invention, but for example, the dye can be synthesized by subjecting boronic acid of a benzene substituted with an alkyl group, an alkoxy group or an amino group, and 5-bromothiophene-2-carboaldehyde to cross-coupling by a Suzuki reaction, and then condensing the aldehyde and cyanoacetic acid by a Knoevenagel reaction. Alternatively, the dye can also be synthesized by subjecting bromide of the substituted benzene described above and 2-thiopheneboronic acid to cross-coupling by a Suzuki reaction, subsequently introducing a formyl group by a Vilsmeier reaction, and finally condensing the aldehyde and cyanoacetic acid by a Knoevenagel reaction.

The various constituent elements of the dye-sensitized solar cell of the present invention will be described below.

The dye-sensitized solar cell of the present invention is configured such that a porous semiconductor layer having sensitizing dyes adsorbed thereon, a carrier transport layer, and a counter electrode are sequentially laminated on a conductive support, and is characterized in that the sensitizing dyes are composed of two different kinds of dyes, and the porous semiconductor is formed of titanium oxide.

### (Regarding conductive support)

The conductive support used in the present invention may be such that the support itself has conductivity as in the case of a metal. Alternatively, a support of glass, plastic or the like having a conductive layer at the surface can also be used. In the case of the latter, preferred examples of the conductive material of the conductive layer include metals such as gold, platinum, silver, copper, aluminum and indium; conductive carbon, indium tin oxide, and fluorine-doped tin oxide, and a conductive layer can be formed on a support by a conventional method using these conductive materials. The film thickness of such a conductive layer is preferably about 0.02 µm to 5 µm. Regarding the conductive support, a support having lower surface resistance is more preferred, and the surface resistance is preferably 40 Ω/sq or less. When the conductive support is used as a light-receiving surface, it is preferable that the support be transparent. Furthermore, the film thickness of the conductive support is not particularly limited as long as appropriate strength can be imparted to the photoelectric conversion layer. When these points and mechanical strength are considered, a conductive layer formed from tin oxide doped with fluorine is laminated on a transparent substrate formed from soda lime float glass, can be used as a representative support.

Furthermore, when cost, flexibility and the like are taken into consideration, a transparent polymer sheet provided thereon with the aforementioned conductive layer may also be used. Examples of the transparent polymer sheet include sheets of tetraacetyl cellulose, polyethylene terephthalate, polyphenylene sulfide, polycarbonate, polyallylate, polyether imide, and phenoxy resins. Furthermore, in order to decrease the resistance of the transparent substrate, a metal lead wire may be added thereto. The material of the metal lead wire is preferably platinum, copper, aluminum, indium, nickel or the like. The metal lead wire is provided on the transparent substrate by sputtering, vapor deposition or the like, and a transparent conductive film of tin oxide, ITO or the like may also be provided thereon.

### (Semiconductor layer)

A porous semiconductor layer is formed from aggregates of nano particles, and regarding the nano particles, any fine particles that are generally used in photoelectric conversion materials can be used. Examples include fine particles of titanium oxide, zinc oxide, tin oxide, iron oxide, niobium oxide, zirconium oxide, cerium oxide, tungsten oxide, silicon oxide, aluminum oxide, nickel oxide, barium titanate, strontium titanate, cadmium sulfide, lead sulfide, zinc sulfide, indium phosphide, and copper indium sulfide, which may be used singly or in combination. Among them, titanium oxide, zinc oxide, tin oxide, and niobium oxide are preferred, and from the viewpoints of stability and safety, titanium oxide is particularly preferred.

In the Examples of the present invention that will be described below, titanium oxide was used as the material of the nano particles. Crystalline titanium oxide has two kinds of crystal types such as anatase type and rutile type, and any of them can be employed according to the production method or thermal history; however, it is general to use a mixture thereof. Regarding the material of the nano particles, anatase type is preferred from the viewpoint of the photocatalytic activity, and even for a mixture, a mixture having a percentage content of the anatase type of 90% or higher is preferred. The anatase type titanium oxide may be a commercially available powder, sol or slurry, or titanium oxide having a predetermined particle size may also be produce according to known methods described in various literatures. There are no particular limitations on the particle size of the nano particles, but a fine powder (average particle size being 1 nm to 1 µm) is preferred. Also, two or more kinds of particles having different particle sizes may be used as a mixture. In this case, it is desirable that the proportions of the average particle sizes be different by 10 or more times. Particles having a large particle size (preferably, 100 nm to 500 nm) have an effect of scattering incident light and thus increasing the quantum yield. Also, particles having a small particle size (preferably 5 nm to 50 nm) have an effect of increasing the number of adsorption points and promoting dye adsorption.

The method for forming a porous semiconductor layer is not particularly limited, and a known method may be used. For example, a method of applying a suspension liquid containing nano particles on a transparent conductive film, and drying and calcining the suspension liquid may be used. Examples of the solvent of suspending nano particles include ethylene glycol monomethyl ether, isopropyl alcohol, an isopropyl alcohol-toluene mixed solvent, and water. Furthermore, a commercially available titanium oxide paste may be used instead of the suspension liquid. Application on the substrate can be carried out by various known methods such as a dipping method, a spraying method, a wire bar method, a spin coating method, a roller coating method, a blade coating method, a gravure coating method, and screen printing. The temperature, time, atmosphere and the like of drying and calcination can be respectively adjusted according to the kinds of the substrate and the nano particles. Usually, drying and calcination is carried out at atmospheric pressure at 40°C to 700°C for about 10 minutes to 10 hours. Furthermore, the processes of coating, drying and calcination may be repeated two or more times.

In order to make the porous semiconductor layer capable of adsorbing a larger amount of dye molecules, a semiconductor layer having a large surface area and a large layer thickness is preferable because the amount of supported dye is increased. However, when this surface area is increased, the diffusion distance of injected electrons is increased. Accordingly, the loss caused by charge recombination is also increased. Therefore, the surface is preferably about 10 m²/g to 200 m²/g, and the thickness is preferably about 0.1 µm to 100 µm.

### (Method for adsorbing sensitizing dyes)

Regarding the method for adsorbing sensitizing dyes to a porous semiconductor, a method of immersing a semiconductor electrode in a solution having sensitizing dyes dissolved therein is generally used. Examples of the solvent for the dye solution include organic solvents such as alcohol, toluene, acetonitrile, tetrahydrofuran, chloroform, and dimethylformamide, and in order to increase solubility, two or more kinds of solvents may be mixed. The dye concentration in the solvent may be appropriately adjusted according to the kind of the sensitizing dye or the solvent, but the dye concentration is preferably about 0.01 mM to 10 mM. Furthermore, if necessary, deoxycholic acid or the like may be added in order to reduce association of the dye molecules. The immersion time is appropriately adjusted according to the kind of the sensitizing dyes and solvent used, the concentration of the solution and the like, but the immersion time is preferably 2 to 50 hours, and the temperature at the time of immersion is preferably 10°C to 50°C. Immersion may be carried out once, or may be carried out several times. Furthermore, when the amount of adsorption of the dyes is large, the dye molecules that are not directly bonded to the semiconductor are released to the carrier transport layer of the solar cell and cause a decrease in the energy conversion efficiency. Therefore, after the semiconductor is immersed in a dye solution, it is preferable to wash the semiconductor with an organic solvent to remove any unadsorbed dyes. Examples of the cleaning agent include methanol, ethanol, acetonitrile and acetone, which are all relatively highly volatile. Furthermore, after excess dyes have been removed by washing, the surface of the semiconductor may be treated with an organic basic compound to accelerate the removal of unadsorbed dyes, in order to make the adsorbed state of the dyes more stable. Examples of the organic basic compound include derivatives of pyridine and quinoline. When these compounds are liquids, the compounds may be used directly, but when they are solids, the compounds may be used in the form of being dissolved in the same solvent as that of the dye solution.

According to the present invention, two kinds of dyes are adsorbed as a mixture onto a porous semiconductor, and examples of the method for adsorption include a method of preparing a dye solution in which two kinds of dyes are dissolved in the same solvent, and immersing a semiconductor electrode therein; and a method of immersing a semiconductor electrode in a dye solution in which one kind of dye is dissolved, and then immersing the semiconductor electrode in a dye solution in which another dye is dissolved. In the latter method of adsorbing dyes separately, it is preferable to initially adsorb a dye having a larger molecular size, and then adsorb a dye having a smaller molecular size.

### (Carrier transport layer)

A carrier transport layer contains a conductive material that can transport electrons, holes and ions. Examples of such a conductive material include hole transporting materials such as polyvinylcarbazole and triphenylamine; electron transporting materials such as tetranitrofluorenone; conductive polymers such as polythiophene and polypyrrole; ion conductors such as liquid electrolytes and polymeric electrolytes; and inorganic P-type semiconductors such as copper iodide and copper thiocyanate.

Among the conductive materials described above, an ion conductor capable of transporting ions is preferred, and a liquid electrolyte containing a redox electrolyte is particularly preferred. Regarding such a redox electrolyte, generally, there are no particular limitations as long as a redox electrolyte can be used in batteries or solar cells. Specific examples include electrolytes containing redox species such as an I⁻/I₃⁻ system, a Br₂1Br₃⁻ system, a Fe₂⁺/Fe₃⁺ system, and a quinone/hydroquinone system. For example, combinations of metal iodides such as lithium iodide, potassium iodide and calcium iodide, with iodine; combinations of tetraalkylammonium salts such as tetraethylammonium iodide, tetrapropylammonium iodide, tetrabutylammonium iodide and tetrahexylammonium iodide, with iodine; and combinations of metal bromides such as lithium bromide, sodium bromide, potassium bromide and calcium bromide, with bromine, are preferred, and among these, a combination of lithium iodide and iodine is particularly preferred.

When a liquid electrolyte is used in the carrier transport layer, examples of the solvent that can be used therein include carbonate compounds such as propylene carbonate; nitrile compounds such as acetonitrile; alcohols such as ethanol; water, and aprotic polar substances. Among these, a carbonate compound or a nitrile compound is particularly preferred. Furthermore, these solvents can be used as mixtures of two or more kinds. Also, the electrolyte concentration in the liquid electrolyte is preferably 0.1 M to 1.5 M, and particularly preferably 0.1 M to 0.7 M.

Furthermore, the liquid electrolyte may contain various additives. Examples of the additives include nitrogen-containing aromatic compounds such as 4-tert-butylpyridine, and imidazolium salts such as dimethylpropylimidazolium iodide, which have been conventionally used. These additives may be added to the liquid electrolyte at a concentration of about 0.1 M to 1.5 M.

### (Counter electrode)

A counter electrode can constitute a pair of electrodes together with a dye-sensitized semiconductor electrode, and usually, the counter electrode is formed such that a conductive layer and a catalyst layer are laminated on a supporting substrate toward a semiconductor electrode side. Examples of the supporting substrate include transparent or opaque substrates that can be used as substrates of solar cells. Examples of the material of the conductive layer include N-type or P-type elemental semiconductors (for example, silicon and germanium); compound semiconductors (for example, GaAs, InP, ZnSe, and CsS); metals such as gold, platinum, silver, copper and aluminum; high melting point metals such as titanium, tantalum and tungsten; and transparent conductive materials such as ITO, SnO₂, Cul and ZnO. These conductive layers can be formed on supporting substrates by conventional methods, and a film thickness of about 0.1 µm to 1.0 µm is appropriate. Examples of the material of the catalyst layer include platinum, carbon black, carbon nanotubes, and fullerene. In the case of platinum, the catalyst layer can be formed on the conductive layer by methods such as sputtering, thermal decomposition of chloroplatinic acid, or electrodeposition. Furthermore, for the purpose of enhancing an effect of catalyzing redox, it is preferable that the side that is in contact with the semiconductor electrode have a microstructure with an increased surface area. Also, for example, if platinum is used, it is preferable that the catalyst layer be in the state of black platinum, and if carbon is used, it is preferable that the catalyst layer be in a porous state.

### (Spacer)

In order to prevent contact between the counter electrode and porous semiconductor layer, a spacer may be inserted therebetween. As the spacer, a polymer film of polyethylene or the like is used. The film thickness of this film is appropriately about 10 µm to 50 µm.

The present invention will be described more specifically by way of the following Examples, but the present invention is not intended to be limited to those.

### Examples

The present invention will be described more specifically by way of the following Examples, but the present invention is not intended to be limited to those.

### [Synthesis Example 1] Synthesis of sensitizing dyes II-1, II-2, II-3 and II-4

### (1) Synthesis of II-1 [2-cyano-3-(5-(2,4-dimethoxyphenyl)thiophen-2-yl)acrylic acid]

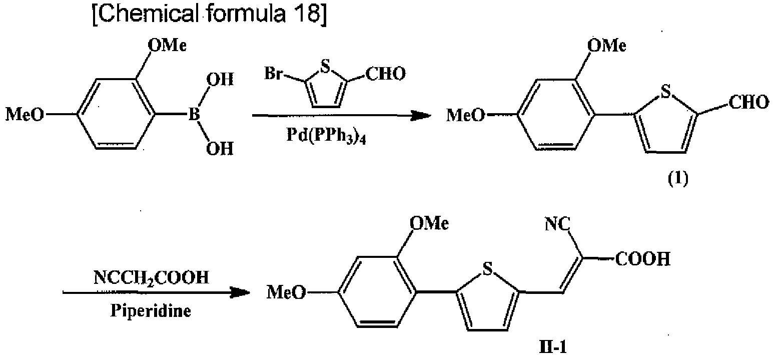

### 5-(2,4-Dimethoxyphenyl)thiophene-2-carbaldehyde (1)

2,4-Dimethoxyphenylboronic acid (972 mg, 5.34 mmol), 5-bromothiophene-2-carboxaldehyde (874 mg, 4.57 mmol), and Pd(PPh₃)₄ (135 mg) are dissolved in a mixed solvent of toluene and ethanol (80 ml/40 ml). An aqueous solution (15 ml) of potassium carbonate (2 g) is added thereto, and the reaction mixture liquid is heated to reflux for 24 hours in an argon atmosphere. Water is added thereto, and the mixture is extracted with dichloromethane. The extract is dried over anhydrous sodium sulfate, and then is concentrated under reduced pressure. The residue is purified by silica gel column chromatography (dichloromethane/hexane = 2/1). Thus, an aldehyde (1) was obtained (1090 mg, 96%).
¹H NMR (600 MHz, CDCl₃): δ9.88 (s, 1H), 7.70 (d, J= 4.2 Hz, 1 H), 7.65 (d, J= 8.4 Hz, 1H), 7.49 (d, J= 4.2 Hz, 1 H), 6.58 (dd, J= 8.4 and 2.4 Hz, 1 H), 6.55 (d, J= 2.4 Hz, 1H), 3.95 (s, 3H), 3.86 (s, 3H).

### 2-Cyano-3-(5-(2,4-dimethoxyphenyl)thiophen-2-yl)acrylic acid (II-1)

The aldehyde (1) (1085 mg, 4.37 mmol) and cyanoacetic acid (372 mg, 4.37 mmol) are dissolved in acetonitrile (50 ml), piperidine (0.35 ml) is added thereto, and the mixture is heated to reflux for 24 hours in an argon atmosphere. The reaction mixture liquid is acidified with 2 N HCl, water is added thereto, and the mixture is extracted with dichloromethane. The extract is dried over anhydrous sodium sulfate, and then is concentrated under reduced pressure. The residue is purified by silica gel column chromatography (dichloromethane → methanol). Thus, II-1 was obtained as a red solid (161 mg, 11%).
¹H NMR (600 MHz, DMSO-d6):δ8.44 (s, 1H), 7.96 (d, J= 4.2 Hz, 1H), 7.86 (d, J= 8.4 Hz, 1H), 7.75 (d, J=4.2 Hz, 1 H), 6.76 (d, J= 2.4 Hz, 1H), 6.70 (dd, J= 8.4 and 2.4 Hz, 1H), 3.97 (s, 3H), 3.86 (s, 3H).
Exact mass calculated for C₁₆H₁₂NO₄S [M-1]⁻314.0493, observed 314.0498. UV/VIS (EtOH): Amax = 393 nm (ε 28,400)

### (2) Synthesis of II-2 [2-cyano-3-(5-(4-methoxyphenyl)thiophen-2-yl)acrylic acid]

Sensitizing dye II-2 was synthesized by the same synthesis method using 4-methoxyphenylboronic acid instead of 2,4-dimethoxyphenylboronic acid of Synthesis Example 1(1) Synthesis Example of sensitizing dye II-1. 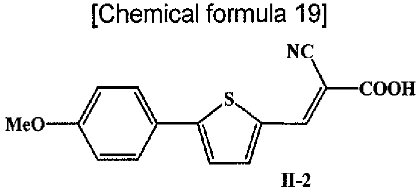 ¹H NMR (600 MHz, DMSO-d6):δ8.47 (s, 1 H), 7.99 (d, J= 3.6 Hz, 1 H), 7.75 (d, J= 9.0 Hz, 2H), 7.66 (d, J= 3.6 Hz, 1 H), 7.06 (d, J= 9.0 Hz, 2H), 3.82 (s, 3H). UV/VIS (EtOH): Amax = 382 nm (ε 32,700)

### (3) Synthesis of II-3 [2-cyano-3-(5-phenylthiophen-2-yl)acrylic acid]

Sensitizing dye II-3 was synthesized by the same synthesis method using phenylboronic acid instead of 2,4-dimethoxyphenylboronic acid of Synthesis Example 1(1) Synthesis Example of sensitizing dye II-1. 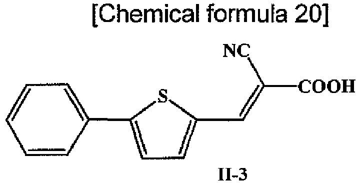 ¹H NMR (600 MHz, DMSO-d6):δ8.51 (s, 1 H), 8.03 (d, J= 4.2 Hz, 1 H), 7.80 (d, J= 7.2 Hz, 2H), 7.78 (d, J= 4.2 Hz, 1H), 7.52 (t, J= 7.2 Hz, 2H), 7.46 (t, J= 7.2 Hz, 1 H). UV/VIS (EtOH): Amax = 365 nm (ε 31,500)

### (4) Synthesis of II-4 [2-cyano-3-(5-(4-dimethylaminophenyl)thiophen-2-yl)acrylic acid]

Sensitizing dye II-4 was synthesized by the same synthesis method using 4-dimethylaminophenylboronic acid instead of 2,4-dimethoxyphenylboronic acid of Synthesis Example 1(1) Synthesis Example of sensitizing dye II-1. 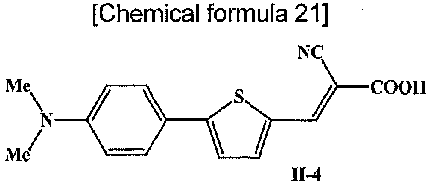 ¹H NMR (600 MHz, DMSO-d6):δ8.41 (s, 1H), 7.94 (d, J= 4.2 Hz, 1H), 7.63 (d, J= 9.0 Hz, 2H), 7.56 (d, J= 4.2 Hz, 1 H), 6.79 (d, J= 9.0 Hz, 2H), 2.99 (s, 6H) UV/VIS (EtOH): Amax = 424 nm (ε 27,600)

### [Synthesis Example 2] Synthesis of sensitizing dyes II-5, II-6, II-7, II-8, and II-9 (1) Synthesis of II-5 [2-cyano-3-(5-(4-octyloxyphenyl)thiophen-2-yl)acrylic acid]

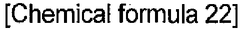

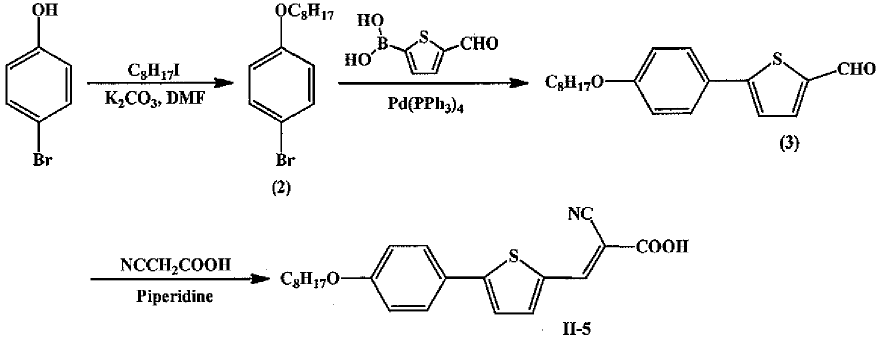

### 5-(4-Octyloxyphenyl)thiophene-2-carbaldehyde (3)

4-Bromophenol (1.0 g, 5.78 mmol), 1-iodooctane (1.67 g, 6.94 mmol), and potassium carbonate (4.0 g, 29 mmol) are dissolved in DMF (40 ml), and the solution is heated to reflux for 24 hours. Water is added to the reaction mixture liquid, and the mixture is extracted with dichloromethane. The extract is dried over anhydrous sodium sulfate, and then is concentrated under reduced pressure. The residue is purified by silica gel column chromatography (dichloromethane/hexane = 1/1). Thus, bromide (2) (1.48 g) was obtained.

The bromide (2) (500 mg, 1.75 mmol), 5-formyl-2-thiopheneboronic acid (230 mg, 1.47 mmol) and PdCl₂ (dppf) (53 mg) are dissolved in a mixed solvent of toluene and methanol (40 ml/20 ml), and an aqueous solution (10 ml) of potassium carbonate (1.5 g) is added thereto. The mixture is heated to reflux for 24 hours in an argon atmosphere. Water is added to the reaction mixture liquid, and the mixture is extracted with dichloromethane. The extract is dried over anhydrous sodium sulfate, and then is concentrated under reduced pressure. The residue is purified by silica gel column chromatography (dichloromethane/hexane =1/1). Thus, aldehyde (3) was obtained (140 mg, 23%).
¹H NMR (600 MHz, CDCl₃):δ9.86 (s, 1H), 7.71 (d, J= 3.6 Hz, 1H), 7.60 (d, J= 9.0 Hz, 2H), 7.29 (d, J= 3.6 Hz, 1 H), 6.94 (d, J= 9.0 Hz, 2H), 4.00 (t, J= 6.6 Hz, 2H), 1.80 (m, 2H), 1.46 (m, 2H), 1.38-1.25 (m, 8H), 0.89 (t, J= 6.6 Hz, 3H).

### 2-Cyano-3-(5-(4-octyloxyphenyl)thiophen-2-yl)acrylic acid (II-5)

The aldehyde (3) (140 mg, 0.44 mmol) and cyanoacetic acid (40 mg, 0.47 mmol) are dissolved in acetonitrile (30 ml), piperidine (0.1 ml) is added thereto. The mixture is heated to reflux for 24 hours in an argon atmosphere. The reaction mixture liquid is acidified with 2 N HCl, water is added thereto, and the mixture is extracted with dichloromethane. The extract is dried over anhydrous sodium sulfate, and then is concentrated under reduced pressure. The residue is purified by silica gel column chromatography (dichloromethane → methanol). Thus, II-5 was obtained as an orange-colored solid (115 mg, 68%).
¹H NMR (600 MHz, DMSO-d6):δ8.47 (s, 1 H), 7.99 (d, J= 3.6 Hz, 1H), 7.73 (d, J= 9.0 Hz, 2H), 7.66 (d, J= 3.6 Hz, 1H), 7.04 (d, J= 9.0 Hz, 2H), 4.03 (t, J= 6.6 Hz, 2H), 1.73 (m, 2H), 1.42 (m, 2H), 1.35-1.29 (m, 8H), 0.86 (t, J= 6.6 Hz, 3H). Exact mass calculated for C₂₂H₂₄NO₃S [M-1]⁻382.1482, observed 382.1488. UV/VIS (EtOH): Amax = 384 nm (ε 30,100)

### (2) Synthesis of II-6 [2-cyano-3-(5-(2,4-dibutoxyphenyl)thiophen-2-yl)acrylic acid]

1-Bromo-2,4-dibutoxybenzene was produced from 4-bromoresorcinol and 1-iodobutane, and sensitizing dye II-6 was synthesized by the same synthesis method as that used in Synthesis Example 2(1) Synthesis Example of sensitizing dye II-5. 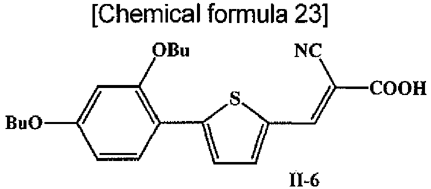 ¹H NMR (600 MHz, DMSO-d6):δ8.16 (s, 1H), 7.73 (d, J= 8.4 Hz, 1H), 7.72 (d, J= 3.6 Hz, 1 H), 7.64 (d, J= 3.6 Hz, 1 H), 6.70 (d, J= 1.8 Hz, 1H), 6.64 (dd, J= 8.4 and 1.8 Hz, 1 H), 4.14 (t, J= 6.6 Hz, 2H), 4.04 (t, J= 6.6 Hz, 2H), 1.87 (m, 2H), 1.71 (m, 2H), 1.53 (m, 2H), 1.45 (m, 2H), 0.96 (t, J= 7.2 Hz, 3H), 0.95 (t, J= 7.2 Hz, 3H). UV/VIS (EtOH): Amax = 396 nm (ε 30,200)

### (3) Synthesis of II-7 [2-cyano-3-(5-(2,4-dioctyloxyphenyl)thiophen-2-yl)acrylic acid]

1-Bromo-2,4-dioctyloxybenzene was produced from 4-bromoresorcinol and 1-iodooctane, and sensitizing dye II-7 was synthesized by the same synthesis method as that used in Synthesis Example 2(1) Synthesis Example of sensitizing dye II-5. 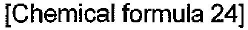 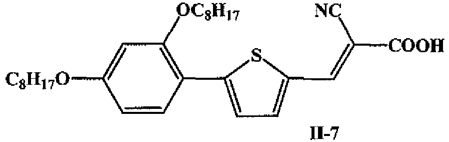 ¹H NMR (600 MHz, CDCl₃):δ8.32 (s, 1 H), 7.84 (d, J= 3.0 Hz, 1H), 7.69 (d, J= 9.0 Hz, 1 H), 7.56 (d, J= 3.0 Hz, 1 H), 6.56 (d, J= 9.0 Hz, 1 H), 6.54 (s, 1H), 4.11 (t, J= 6.0 Hz, 2H), 4.00 (t, J= 6.0 Hz, 2H), 1.99 (m, 2H), 1.81 (m, 2H), 1.53 (m, 2H), 1.49 (m, 2H), 1.43-1.23 (m, 16H), 0.88 (m, 6H).
Exact mass calculated for C₃₀H₄₀NO₄S [M-1]⁻ 510.2684, observed 510.2690. UV/VIS (EtOH): λmax = 395 nm (ε26,100)

### (4) Synthesis of II-8 [3-(5-(2,4-bis(hexadecyloxy)phenyl)thiophen-2-yl)-2-cyanoacrylic acid]

1-Bromo-2,4-bis(hexadecyloxy)benzene was produced from 4-bromoresorcinol and 1-iodohexadecane, and sensitizing dye II-8 was synthesized by the same synthesis method as that used in Synthesis Example 2(1) Synthesis Example of sensitizing dye II-5. 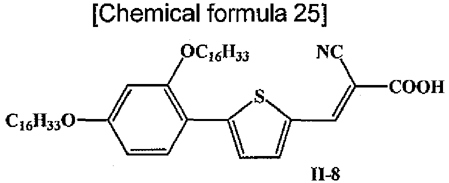 ¹H NMR (600 MHz, CDCl₃):δ8.26 (s, 1 H), 7.77 (d, J= 4.2 Hz, 1 H), 7.66 (d, J= 9.0 Hz, 1 H), 7.52 (d, J= 4.2 Hz, 1H), 6.54 (d, J= 9.0 Hz, 1 H), 6.52 (s, 1H), 4.09 (t, J= 6.6 Hz, 2H), 3.99 (t, J= 6.6 Hz, 2H), 1.96 (m, 2H), 1.80 (m, 2H), 1.51 (m, 2H), 1.46 (m, 2H), 1.40-1.20 (m, 48H), 0.88 (m, 6H).
Exact mass calculated for C₄₆H₇₂NO₄S [M-1]⁻734.5188, observed 734.5198. UV/VIS (EtOH): λmax = 392 nm (ε 26,900)

### (5) Synthesis of II-9 [3-(5-(4-butoxyphenyl)thiophen-2-yl)-2-cyanoacrylic acid]

Sensitizing dye II-9 was synthesized from 1-bromo-4-butoxybenzene by the same synthesis method as that used in Synthesis Example 2(1) Synthesis Example of sensitizing dye II-5. 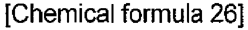 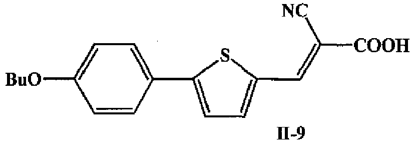 ¹H NMR (600 MHz, DMSO-d6):δ8.23 (s, 1 H), 7.80 (d, J= 4.2 Hz, 1 H), 7.68 (d, J= 9.0 Hz, 2H), 7.57 (d, J= 4.2 Hz, 1 H), 7.03 (d, J= 9.0 Hz, 2H), 4.02 (t, J= 6.6 Hz, 2H), 1.71 (qui, J= 6.6 Hz,2H), 1.45 (sex, J= 7.2 Hz, 2H), 0.94 (t, J= 7.2 Hz, 3H).
Exact mass calculated for C₁₈H₁₆NO₃S [M-1]⁻ 326.0856, observed 326.0859. UV/VIS (DMSO): Amax = 377 nm (ε 25,600)

### [Synthesis Example 3] Synthesis of sensitizing dyes II-10 and II-11

### (1) Synthesis of II-10 [2-cyano-3-(5-(4-dibutylaminophenyl)thiophen-2-yl)acrylic acid]

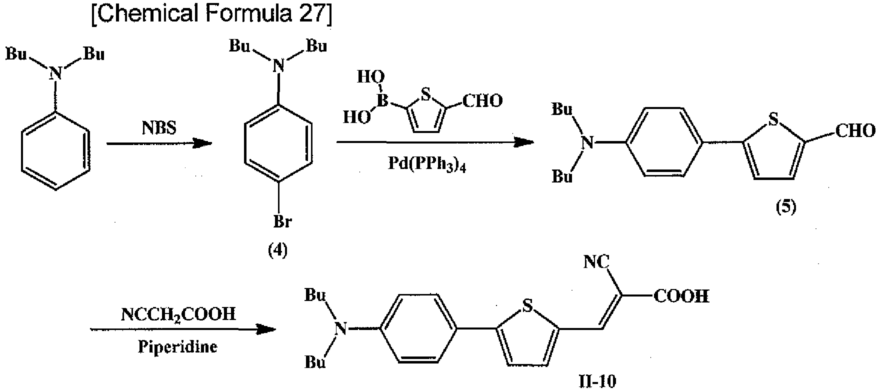

### 5-(4-Dibutylaminophenyl)thiophene-2-carbaldehyde (5)

NBS (4.3 g, 24.3 mmol) is added to a DMF (50 ml) solution of N,N-dibutylaniline (5.0 g, 24.3 mmol), and the mixture is stirred for 1.5 hours at room temperature. Water is added to the reaction solution, and the reaction product is extracted with dichloromethane. The extract is dried over anhydrous sodium sulfate, and then is concentrated under reduced pressure. The residue is purified by silica gel column chromatography (hexane). Thus, bromide (4) (6.17 g) was obtained as an oily matter.

The bromide (4) (1.5 g, 5.28 mmol), 5-formyl-2-thiopheneboronic acid (0.69 g, 4.4 mmol) and Pd(PPh₃)₄ (150 mg) are dissolved in a mixed solvent of toluene and ethanol (80 ml/40 ml), and an aqueous solution (15 ml) of potassium carbonate (2 g) is added thereto. The mixture is heated to reflux for 24 hours in an argon atmosphere. Water is added to the reaction mixture liquid, and the mixture is extracted with dichloromethane. The extract is dried over anhydrous sodium sulfate, and then is concentrated under reduced pressure. The residue is purified by silica gel column chromatography (dichloromethane/hexane =1/1). Thus, aldehyde (5) was obtained (424 mg, 28%).
¹H NMR (600 MHz, CDCl₃):δ9.81 (s, 1H), 7.77 (d, J = 4.2 Hz, 1 H), 7.53 (d, J = 9.0 Hz, 2H), 7.21 (d, J = 4.2 Hz, 1 H), 6.64 (d, J = 9.0 Hz, 2H), 3.31 (t, J = 7.2 Hz, 4H), 1.57 (m, 4H), 1.38 (m, 4H), 0.97 (t, J = 7.2 Hz, 6H).

### 2-Cyano-3-(5-(4-dibutylaminophenyl)thiophen-2-yl)acrylic acid (II-10)

The aldehyde (5) (424 mg, 1.34 mmol) and cyanoacetic acid (114 mg, 1.34 mmol) are dissolved in acetonitrile (30 ml), piperidine (0.1 ml) is added thereto, and the mixture is heated to reflux for 24 hours in an argon atmosphere. The reaction mixture liquid is acidified with 2 N HCl, water is added thereto, and the mixture is extracted with dichloromethane. The extract is dried over anhydrous sodium sulfate, and then is concentrated under reduced pressure. The residue is purified by silica gel column chromatography (dichloromethane → methanol). Thus, II-10 was obtained as a dark red solid (148 mg, 29%).
¹H NMR (600 MHz, DMSO-d6):δ8.39 (s, 1 H), 7.92 (d, J= 4.2 Hz, 1 H), 7.58 (d, J= 9.0 Hz, 2H), 7.51 (d, J= 4.2 Hz, 1 H), 6.71 (d, J= 9.0 Hz, 2H), 3.34 (t, J= 7.2 Hz, 4H), 1.52 (m, 4H), 1.33 (m, 4H), 0.92 (t, J= 7.2 Hz, 6H).
Exact mass calculated for C₂₂H₂₅N₂O₂S [M-1]⁻ 381.1642, observed 381.1649. UV/VIS (EtOH): λmax = 438 nm (ε20,500)

### (2) Synthesis of II-11 [2-cyano-3-(5-(4-dioctylaminophenyl)thiophen-2-yl)acryiic acid]

N,N-dioctylaniline was produced from aniline and 1-iodooctane, and sensitizing dye II-11 was synthesized by the same synthesis method as that used in Synthesis Example 3(1) Synthesis Example of sensitizing dye II-10. 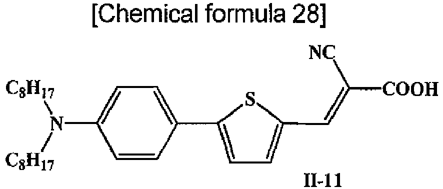 ¹H NMR (600 MHz, CDCl₃):δ8.28 (s, 1 H), 7.72 (d, J= 4.2 Hz, 1 H), 7.58 (d, J= 9.0 Hz, 2H), 7.27 (d, J= 4.2 Hz, 1H), 6.63 (d, J= 9.0 Hz, 2H), 3.32 (t, J= 7.2 Hz, 4H), 1.61 (m, 4H), 1.42-1.22 (m, 20H), 0.89 (t, J= 7.2 Hz, 6H).
Exact mass calculated for C₃₀H₄₁N₂O₂S [M-1]⁻ 493.2894, observed 493.2901. UV/VIS (EtOH): Amax = 438 nm (ε31,600)

### [Synthesis Example 4] Synthesis of sensitizing dyes II-12, II-13 and II-14

### (1) Synthesis Example of II-12 [2-cyano-3-(5-(4-dibutylaminophenyl)-4-octylthiophen-2-yl)acrylic acid]

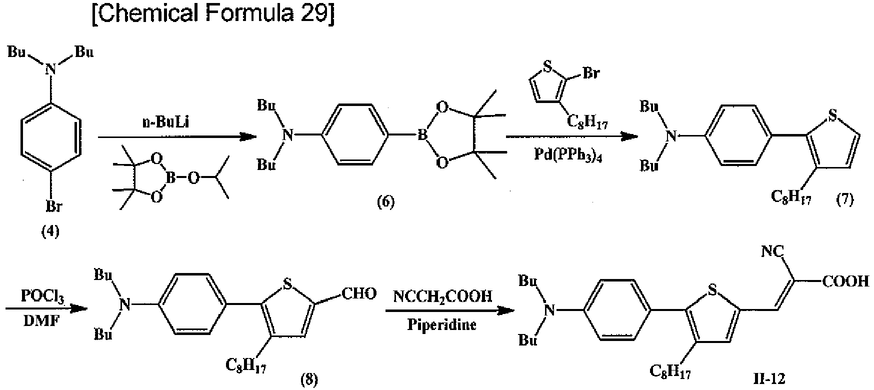

### 5-(4-Dibutylaminophenyl)-4-octylthiophene-2-carbaldehyde (8)

The bromide (4) (1000 mg, 3.52 mmol) is dissolved in anhydrous THF (100 ml) in an argon atmosphere, the solution is cooled to -78°C, and then a 15% hexane solution of n-butyllithium (2.6 ml, 4.22 mmol) is added dropwise thereto. After completion of dropwise addition, the mixture is stirred for another 2 hours at - 78°C. 2-Isopropoxy-4,4,5,5-tetramethyl-1,3,2-dioxaborolane (851 mg, 4.58 mmol) is added dropwise thereto, and while stirring is continued for 24 hours, the reaction temperature is returned to room temperature. Water is added to the reaction mixture liquid, and the mixture is extracted with ethyl acetate. The extract is dried over sodium sulfate, and then is concentrated under reduced pressure to obtain boronic acid (6) (1095 mg, 3.3 mmol). This boronic acid (6), 2-bromo-3-octylthiophene (1092 mg, 3.97 mmol), and Pd(PPh₃)₄ (114 mg) are dissolved in toluene (100 ml), an aqueous solution (20 ml) of potassium carbonate (5 g) is added thereto, and the mixture is heated to reflux for 24 hours in an argon atmosphere. Water is added to the reaction mixture liquid, and the mixture is extracted with dichloromethane. The extract is dried over anhydrous sodium sulfate, and then is concentrated under reduced pressure. The residue is purified by silica gel column chromatography (dichloromethane/hexane = 1/2). Thus, thiophene (7) (900 mg, 2.25 mmol) was obtained. This thiophene (7) is dissolved in DMF, the solution is cooled to 0°C, and phosphorus oxychloride (863 mg, 5.63 mmol) is added dropwise thereto. The mixture is stirred for 4 hours at 70°C in an argon atmosphere. The reaction mixture liquid is neutralized with 2 N NaOH, and the mixture is extracted with dichloromethane. The extract is dried over anhydrous sodium sulfate, and then is concentrated under reduced pressure. The residue is purified by silica gel column chromatography (dichloromethane/hexane = 1/1). Thus, aldehyde (8) was obtained (720 mg, 43%).
¹H NMR (600 MHz, An-d6):δ9.85 (s, 1 H), 7.84 (s, 1 H), 7.35 (d, J= 8.4 Hz, 2H), 6.78 (d, J= 8.4 Hz, 2H), 3.40 (t, J= 7.8 Hz, 4H), 2.74 (t, J= 7.8 Hz, 2H), 1.67 (m, 2H), 1.62 (m, 4H), 1.44~1.22 (m, 14H), 0.97 (t, J= 7.2 Hz, 6H), 0.87 (t, J= 7.2 Hz, 3H).

### 2-Cyano-3-(5-(4-dibutylaminophenyl)-4-octylthiophen-2-yl)acrylic acid (II-12)

The aldehyde (8) (720 mg, 1.68 mmol) and cyanoacetic acid (143 mg, 1.68 mmol) are dissolved in acetonitrile (30 ml), piperidine (0.1 ml) is added thereto, and the mixture is heated to reflux for 24 hours in an argon atmosphere. 2 N HCl is added to the reaction solution, subsequently water is added thereto, and the mixture is extracted with dichloromethane. The extract is dried over anhydrous sodium sulfate, and then is concentrated under reduced pressure. The residue is purified by silica gel column chromatography (dichloromethane → methanol). Thus, II-12 as a red brown solid was obtained (580 mg, 70%).
¹H NMR (600 MHz, CDCl₃):δ8.24 (s, 1 H), 7.63 (s, 1 H), 7.35 (d, J= 7.8 Hz, 2H), 6.65 (d, J= 7.8 Hz, 2H), 3.31 (t" J= 7.2 Hz, 4H), 2.69 (m, 2H), 1.60 (m, 6H), 1.38 (m, 4H), 1.26 (m, 10H), 0.98 (t, J= 7.2 Hz, 6H), 0.87 (t, J= 7.2 Hz, 3H). Exact mass calculated for C₃₀H₄₁N₂O₂S [M-1]⁻493.2894, observed 493.2904. UV/VIS (EtOH): λmax = 280, 412 nm (ε 16,000, 18,500)

### (2) Synthesis of II-13 [2-cyano-3-(2-(2,4-dibutoxyphenyl)thiazol-5-yl)acrylic acid]

Sensitizing dye II-13 was synthesized from 1-bromo-2,4-dibutoxybenzene and 2-bromothiazole, by the same synthesis method as that used in Synthesis Example 4(1) Synthesis Example of sensitizing dye II-12. 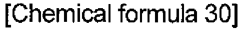 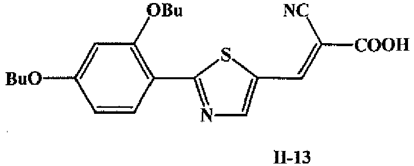 ¹H NMR (600 MHz, DMSO-d6):δ8.33 (s, 1 H), 8.25 (d, J= 9.0 Hz, 1H), 8.20 (s, 1 H), 6.76 (d, J= 1.8 Hz, 1 H), 6.71 (dd, J= 9.0 and 1.8 Hz, 1H), 4.24 (t, J= 6.0 Hz, 2H), 4.08 (t, J= 6.6 Hz, 2H), 1.91 (m, 2H), 1.73 (m, 2H), 1.58 (m, 2H), 1.46 (m, 2H), 0.97 (t, J= 7.2 Hz, 3H), 0.95 (t, J= 7.2 Hz, 3H).
Exact mass calculated for C₂₁H₂₃N₂O₄S [M-1]⁻ 399.1384, observed 399.1389. UV/VIS (EtOH): Amax = 386 nm (ε18,200)

### (3) Synthesis of II-14 [2-cyano-3-(5-(2,4-dibutoxyphenyl)-4-octylthiophen-2-yl)acrylic acid]

Sensitizing dye II-14 was synthesized from 1-bromo-2,4-dibutoxybenzene by the same synthesis method as that used in Synthesis Example 4(1) Synthesis Example of sensitizing dye II-12. 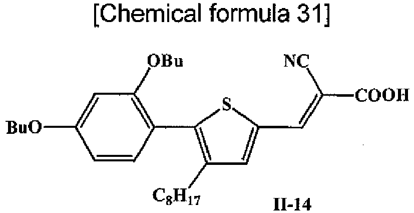 ¹H NMR (600 MHz, DMSO-d6):δ8.31 (s, 1 H), 7.79 (s, 1 H), 7.19 (d, J= 8.4 Hz, 1H), 6.67 (d, J= 1.8 Hz, 1 H), 6.60 (dd, J= 8.4 and 1.8 Hz, 1H), 4.02 (t, J= 6.6 Hz, 2H), 3.99 (t, J= 6.6 Hz, 2H), 2.44 (t, J=7.8 Hz, 2H), 1.72 (m, 2H), 1.61 (m, 2H), 1.46 (m, 4H), 1.35 (m, 2H), 1.21 (m, 2H), 1.15 (m, 8H), 0.95 (t, J= 7.2 Hz, 3H), 0.85 (t, J= 7.2 Hz, 3H), 0.83 (t, J= 7.2 Hz, 3H).
Exact mass calculated for C₃₀H₄₀NO₄S [M-1]⁻ 510.2684, observed 510.2687. UV/VIS (EtOH): λmax = 360 nm (ε23,200)

### [Synthesis Example 5] Synthesis of sensitizing dye II-15 [2-cyano-3-(5-(2,4,6-trimethoxyphenyl)thiophen-2-yl)acrylic acid]

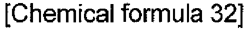

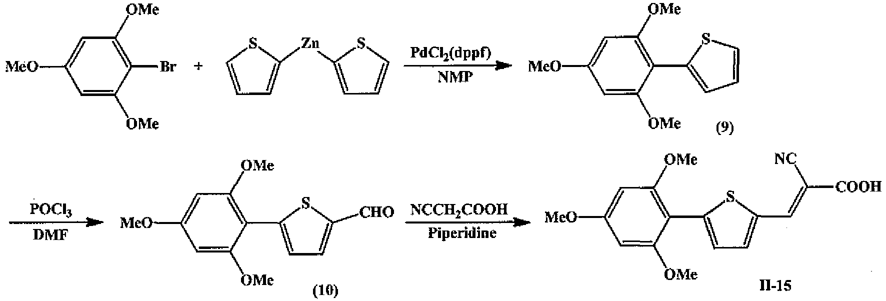

### 5-(2,4,6-Trimethoxyphenyl)thiophene (9)

A solution obtained by dissolving zinc chloride (136 mg, 1.12 mmol) in anhydrous THF (20 ml) is stirred at room temperature in an argon atmosphere, and a 1.0 M THF solution of 2-thienylmagnesium bromide (419 mg, 2.24 mmol) is added dropwise to the solution. After completion of the dropwise addition, the mixture is stirred for another 30 minutes at room temperature, and a dithiophen-2-ylzinc solution is prepared. In that solution, a solution obtained by dissolving 2-bromo-1,3,5-trimethoxybenzene (394 mg, 1.59 mmol), 1-methyl-2-pyrrolidone (3 ml) and PdCl₂ (dppf) (39 mg) in anhydrous dioxane (20 ml), is added dropwise. The reaction mixture liquid is heated to reflux for 24 hours in an argon atmosphere. 1 N HCl is added to the reaction mixture liquid, and then water is added thereto. The mixture is extracted with dichloromethane, and the extract is dried over anhydrous sodium sulfate and then concentrated under reduced pressure. The residue is purified by silica gel column chromatography (dichloromethane/hexane =1/1). Thus, a coupling product (9) was obtained (132 mg, 47%).
¹H NMR (600 MHz, CDCl₃): δ 7.33 (dd, J= 5.4 and 1.2 Hz, 1 H), 7.30 (dd, J= 3.6 and 1.2 Hz, 1 H), 7.07 (dd, J= 5.4 Hz, 3.6Hz, 1 H), 6.22 (s, 2H), 3.85 (s, 3H), 3.81 (s, 6H).

### 2-Cyano-3(5-(2,4,6-trimethoxyphenyl)thiophen-2-yl)acrylic acid (II-15)

A DMF (10 ml) solution of the coupling product (9) (132 mg, 0.53 mmol) is cooled to 0°C, and phosphorus oxychloride (201 mg, 1.31 mmol) is slowly added thereto. The reaction mixture liquid is heated for 2 hours at 70°C in an argon atmosphere. The reaction mixture liquid is neutralized with 1 N NaOH, water is added thereto, and the mixture is extracted with dichloromethane. The extract is dried over anhydrous sodium sulfate, and then is concentrated under reduced pressure. Thus, aldehyde (10) (140 mg) was obtained. This aldehyde and cyanoacetic acid (42 mg, 0.50 mmol) are dissolved in acetonitrile (30 ml), piperidine (0.1 ml) is added thereto, and the mixture is heated to reflux for 24 hours in an argon atmosphere. The reaction mixture liquid is acidified with 2 N HCl, and then water is added thereto. The mixture is extracted with dichloromethane, and the extract is dried over anhydrous sodium sulfate and then concentrated under reduced pressure. The residue is purified by silica gel column chromatography (dichloromethane → methanol). Thus, II-15 was obtained as a red solid (123 mg, 67%).
¹H NMR (600 MHz, DMSO-d6): δ 8.40 (s, 1 H), 7.91 (d, J= 4.2 Hz, 1 H), 7.65 (d, J= 4.2 Hz, 1 H), 6.40 (s, 2H), 3.86 (s, 9H).
Exact mass calculated for C₁₇H₁₄NO₅SNa [M+Na]⁺ 368.0569 , observed 368.0559. UV/VIS (EtOH): Amax = 389 nm (ε18,700)

### [Synthesis Example 6] Synthesis of sensitizing dye II-16 [2-(5-((5-(2,4-dibutoxyphenyl)thiophen-2-yl)methylene)-4-oxo-2-thioxothiazolidin-3-yl)acetic acid]

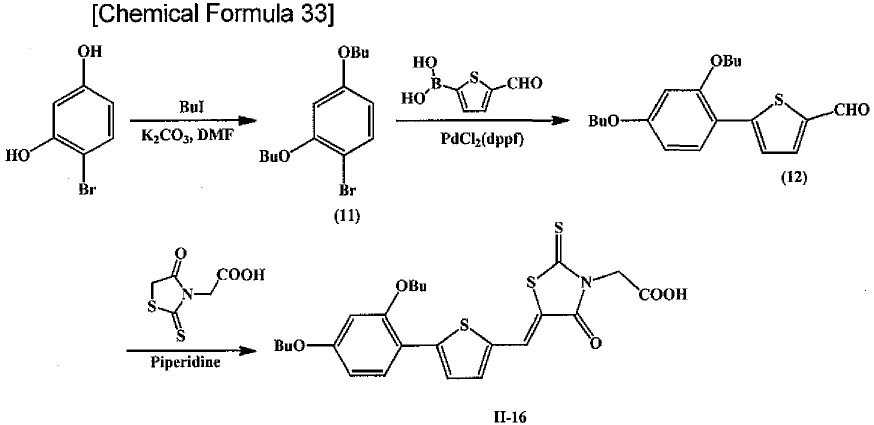

### 5-(2,4-Dibutoxyphenyl)thiophene-2-carbaldehyde (12)

4-Bromoresorcinol (2.0 g, 10.6 mmol), 1-iodobutane (5.1 g, 27.5 mmol), and potassium carbonate (16 g, 58 mmol) are dissolved in DMF (80 ml), and the solution is heated to reflux for 24 hours in an argon atmosphere. Water is added to the reaction mixture liquid, and the mixture is extracted with dichloromethane. The extract is dried over sodium sulfate, and then is concentrated under reduced pressure. The residue is purified by silica gel column chromatography (hexane/dichloromethane = 2/1). Thus, bromide (11) (3.0 g) was obtained as an oily matter.

The bromide (II) (694 mg, 2.31 mmol), 5-formyl-2-thiopheneboronic acid (300 mg, 1.92 mmol), and PdCl₂ (dppf) (70 mg) are dissolved in a mixed solvent of toluene and methanol (40 ml/20 ml), and an aqueous solution (10 ml) of potassium carbonate (2 g) is added thereto. The mixture is heated to reflux for 24 hours in an argon atmosphere. Water is added to the reaction solution, and the mixture is extracted with dichloromethane. The extract is dried over anhydrous sodium sulfate, and then is concentrated under reduced pressure. The residue is purified by silica gel column chromatography (dichloromethane/hexane = 1/1). Thus, aldehyde (12) was obtained (370 mg, 58%).
¹H NMR (600 MHz, CDCl₃): δ 9.88 (s, 1H), 7.70 (d, J= 4.2 Hz, 1 H), 7.63 (d, J= 7.8 Hz, 1H), 7.49 (d, J= 4.2 Hz, 1 H), 6.55 (d, J= 7.8 Hz, 1H), 6.55 (s, 1 H), 4.11 (t, J= 6.6 Hz, 2H), 4.01 (t, J= 6.6 Hz, 2H), 1.92 (m, 2H), 1.79 (m, 2H), 1.56 (m, 2H), 1.52 (m, 2H), 1.00 (t, J= 7.8 Hz, 3H), 0.99 (t, J= 7.8 Hz, 3H).

### 2-(5-((5-(2,4-Dibutoxyphenyl)thiophen-2-yl)methylene)-4-oxo-2-thioxothiazolidin-3-yl)acetic acid (II-16)

The aldehyde (12) (310 mg, 0.93 mmol) and rhodanine-3-acetic acid (178 mg, 0.93 mmol) are dissolved in acetonitrile (30 ml), piperidine (0.1 ml) is added thereto, and the mixture is heated to reflux for 24 hours in an argon atmosphere. The reaction mixture liquid is acidified with 2 N HCl, water is added thereto, and the mixture is extracted with dichloromethane. The extract is dried over sodium sulfate, and then is concentrated under reduced pressure. The residue is purified by silica gel column chromatography (dichloromethane → methanol). Thus, II-16 as a red solid was obtained (370 mg, 78%).
¹H NMR (600 MHz, DMSO-d6):δ8.14 (s, 1 H), 7.90 (d, J= 9.0 Hz, 1H), 7.80 (d, J= 4.2 Hz, 1 H), 7.77 (d, J= 4.2 Hz, 1H), 6.72 (d, J= 2.4 Hz, 1 H), 6.65 (dd, J= 9.0 and 2.4 Hz, 1 H), 4.69 (s, 1H), 4.21 (t, J= 6.0 Hz, 2H), 4.05 (t, J= 6.6 Hz, 2H), 1.95 (m, 2H), 1.73-1.68 (m, 4H), 1.45 (q, J= 7.2 Hz, 2H), 1.07 (t, J= 7.2 Hz, 3H), 0.95 (t, J= 7.2 Hz, 3H).
Exact mass calculated for C₂₄H₂₆NO₅S₃[M-1]⁻ 504.0979, observed 504.0985.
UV/VIS (EtOH): λmax = 467 nm (ε 41,200)

Meanwhile, in general formula (1), the acceptor site is fixed to cyanoacrylic acid, but in the dye II-16, as can be seen from the chemical structural formula described above, the acceptor site is rhodanineacetic acid. As such, even though the cyanoacrylic acid of general formula (1) is replaced with rhodanineacetic acid, the dye exhibits high conversion efficiency as a result of combination with another dye, as indicated in the following Table 1.

### [Example 1]

### Production of porous semiconductor layer

On the transparent conductive side of a SnO₂ film-attached glass plate manufactured by Nippon Sheet Glass Co., Ltd., a commercially available titanium oxide paste (manufactured by Solaronix SA, Ti nanoxide T/SP) was applied by screen printing on the transparent conductive film to a film thickness of about 20 µm and an area of about 5 mm × 5 mm. Furthermore, PST400 manufactured by CCIC Co. was applied thereon (5 µm). The coating film thus obtained was preliminarily dried for 30 minutes at 100°C, and then was calcined for 2 hours at 500°C in an air atmosphere. Thus, a titanium oxide film having a film thickness of 25 µm was obtained as a porous semiconductor layer.

### Adsorption of sensitizing dyes

Purified black dye (BD) (purity 99%) as a sensitizing dye I was dissolved in ethanol at a concentration of 2 × 10⁻⁴ M, and the sensitizing dye II-1 as a sensitizing dye II was dissolved therein at a concentration of 2 × 10⁻⁴ M. To this solution, deoxycholic acid was added and dissolved at a concentration of 2 × 10⁻² M, and thus a solution for mixed adsorption of the sensitizing dye I and the sensitizing dye II was prepared. The aforementioned glass plate was immersed in this solution for 24 hours, and thus, the dyes were adsorbed to the porous semiconductor layer.

### Production of solar cell

A solar cell whose structure is schematically illustrated in Fig. 1, was produced. Specifically, first, a platinum film having a thickness of 1 µm was deposited as a counter electrode conductive layer 6 on a supporting substrate 5, which was a glass substrate including a transparent conductive film, and thereby, a counter electrode 9 composed of the supporting substrate 5 and the counter electrode conductive layer 6 was formed. This counter electrode 9 was arranged to face with a semiconductor electrode formed from a porous semiconductor layer 3 having the aforementioned dyes adsorbed thereon, a transparent conductive film 2 and a supporting substrate 1, and the electrodes were superimposed, with a thermocompressed film spacer for preventing short circuit interposed therebetween, and were tightly sealed. Thus, a member represented as a leakage preventing agent 7 in the diagram was formed. Thereafter, an acetonitrile solution of 1,2-dimethyl-3-propylimidazolium (0.6 M), lithium iodide (0.1 M), iodine (0.05 M) and 4-tert-butylpyridine (0.5 M) as an electrolyte solution was injected into the gap between the two electrodes to form a carrier transport layer 4. Thus, a solar cell was produced.

The solar cell thus obtained was irradiated with light (AM1.5, Solar Simulator) at an intensity of 100 mWcm⁻², and the current-voltage characteristics were measured.

The results of the photoelectric conversion characteristics (short circuit current density (Jsc), open circuit voltage (Voc), fill factor (FF), and energy conversion efficiency) thus obtained are presented in Table 1A.

### [Example 2]

Solar cells were produced in the same manner as in Example 1, using the sensitizing dyes II-2 to II-16, and compounds of formula (6), formula (7), formula (8), formula (10), formula (12), formula (14), and formula (15) as the sensitizing dye II, and the photoelectric conversion characteristics were measured. The results are presented in Table 1A.

### [Comparative Example 1]

For the sensitizing dyes, purified black dye (BD) (purity 99%) was dissolved in ethanol at a concentration of 2 × 10⁻⁴ M, and deoxycholic acid was dissolved therein at a concentration of 2 × 10⁻² M. Thus, a solution for adsorption was prepared. The glass plate of a porous semiconductor layer produced in the same manner as in Example 1 was immersed in the solution for adsorption described above to adsorb the dyes. Thus, a solar cell was produced in the same manner as in Example 1, and the photoelectric conversion characteristics were measured. The results are presented in Table 1B.

### [Comparative Example 2]

Solar cells were produced in the same manner as in Comparative Example 1, using the sensitizing dyes II-1 to II-16 and compounds of formula (6), formula (7), formula (8), formula (10), formula (12), formula (14) and formula (15) as the sensitizing dyes, and the photoelectric conversion characteristics were measured. The results are presented in Table 1B.

**[Table 1A]**

| Dye | Jsc (mA/cm²) | Voc (V) | FF (%) | Energy conversion efficiency (%) |
|---|---|---|---|---|
| BD + II-1 | 21.15 | 0.745 | 0.744 | 11.72 |
| BD + II-2 | 21.01 | 0.751 | 0.745 | 11.75 |
| BD + II-3 | 20.95 | 0.750 | 0.742 | 11.66 |
| BD + II-4 | 21.30 | 0.750 | 0.742 | 11.85 |
| BD + II-5 | 20.70 | 0.749 | 0.734 | 11.38 |
| BD + II-6 | 21.25 | 0.754 | 0.743 | 11.90 |
| BD + II-7 | 21.26 | 0.759 | 0.746 | 12.04 |
| BD + II-8 | 21.25 | 0.760 | 0.744 | 12.02 |
| BD + II-9 | 21.36 | 0.754 | 0.744 | 11.98 |
| BD + II-10 | 21.34 | 0.748 | 0.735 | 11.73 |
| BD + II-11 | 21.72 | 0.753 | 0.743 | 12.15 |
| BD + II-12 | 21.82 | 0.752 | 0.742 | 12.18 |
| BD + II-13 | 21.24 | 0.758 | 0.744 | 11.98 |
| BD + II-14 | 21.22 | 0.748 | 0.743 | 11.79 |
| BD + II-15 | 21.70 | 0.755 | 0.744 | 12.19 |
| BD + II-16 | 21.73 | 0.745 | 0.742 | 12.01 |
| BD + Formula (6) | 22.30 | 0.732 | 0.733 | 11.97 |
| BD + Formula (7) | 21.82 | 0.728 | 0.735 | 11.68 |
| BD + Formula (8) | 21.85 | 0.727 | 0.732 | 11.63 |
| BD + Formula (10) | 23.10 | 0.734 | 0.732 | 12.41 |
| BD + Formula (12) | 21.85 | 0.735 | 0.740 | 11.88 |
| BD + Formula (14) | 22.45 | 0.738 | 0.732 | 12.13 |
| BD + Formula (15) | 22.67 | 0.745 | 0.737 | 12.45 |

**[Table 1B]**

| Dye | Jsc (mA/cm²) | Voc (V) | FF (%) | Energy conversion efficiency (%) |
|---|---|---|---|---|
| BD | 20.50 | 0.720 | 0.720 | 10.63 |
| II - 1 | 5.83 | 0.728 | 0.743 | 3.16 |
| II - 2 | 3.95 | 0.721 | 0.731 | 2.08 |
| II - 3 | 1.69 | 0.730 | 0.740 | 0.92 |
| II - 4 | 8.21 | 0.724 | 0.721 | 4.29 |
| II - 5 | 3.73 | 0.712 | 0.742 | 1.97 |
| II - 6 | 6.23 | 0.720 | 0.733 | 3.29 |
| II - 7 | 5.24 | 0.713 | 0.740 | 2.77 |
| II - 8 | 5.79 | 0.718 | 0.736 | 3.06 |
| II - 9 | 4.65 | 0.732 | 0.744 | 2.54 |
| II - 10 | 9.72 | 0.727 | 0.725 | 5.12 |
| II - 11 | 11.37 | 0.733 | 0.754 | 6.29 |
| II - 12 | 11.05 | 0.729 | 0.761 | 6.13 |
| II - 13 | 5.16 | 0.734 | 0.745 | 2.82 |
| II - 14 | 3.26 | 0.717 | 0.730 | 1.71 |
| II - 15 | 6.10 | 0.719 | 0.750 | 3.29 |
| II - 16 | 7.91 | 0.723 | 0.753 | 4.31 |
| Formula (6) | 10.33 | 0.690 | 0.700 | 4.99 |
| Formula (7) | 8.54 | 0.700 | 0.710 | 4.24 |
| Formula (8) | 11.32 | 0.660 | 0.640 | 4.78 |
| Formula (10) | 11.52 | 0.733 | 0.743 | 6.28 |
| Formula (12) | 11.43 | 0.660 | 0.700 | 5.28 |
| Formula (14) | 8.45 | 0.690 | 0.702 | 4.09 |
| Formula (15) | 8.85 | 0.725 | 0.745 | 4.78 |

From the results of Tables 1A and 1 B, in the dye-sensitized solar cells in which black dye and a dye selected as an organic dye satisfying particular conditions were adsorbed as a mixture, the energy conversion efficiency is dramatically enhanced, as compared with a dye-sensitized solar cell having black dye alone adsorbed thereon. As main factors for this, not only a current density but also an increase in the open circuit voltage has contribution thereto. Furthermore, an organic dye represented by general formula (1) exhibits a higher open circuit voltage, and a compound in which a long-chained alkoxy group and a dialkylamino group are bonded to the benzene ring of the donor site exhibits a high open circuit voltage and excellent conversion efficiency. In this regard, it is speculated that since the organic dye having a smaller molecular size is absorbed so as to be embedded in the gaps formed between dye molecules, which are formed by black dye having a larger molecular size adsorbed to the semiconductor surface, and thereby, the area that is not covered with dyes on the semiconductor surface is reduced, a recombination in which excited electrons injected from the dyes to the semiconductor flow to the carrier transport layer that is in contact with the semiconductor surface, is suppressed. Also, the electrolyte cannot approach the semiconductor surface through the barrier between the semiconductor surface and the carrier transport layer, which is formed by hydrophobic alkyl groups, and the effect by which a recombination is suppressed has increased the open circuit voltage and has thereby dramatically increased the energy conversion efficiency. Furthermore, an organic dye having a large molecular weight and having an alkyl side chain in the molecule exhibits a high open circuit voltage. In this regard, it is speculated that the alkyl side chain has an effect of suppressing association between dye molecules, and at the same time, preventing the electrolyte from infiltrating to the semiconductor surface by means of the hydrophobic layer formed by the alkyl groups, as explained above.

### [Industrial Applicability]

As discussed above in detail, according to the present invention, a dye-sensitized solar cell having a high energy conversion efficiency that has been unknown hitherto can be obtained. Therefore, the present invention has enormous industrial contribution.

### [Reference Signs List]

- 1: supporting substrate
- 2: transparent conductive film
- 3: porous semiconductor layer
- 4: carrier transport layer
- 5: supporting substrate
- 6: counter electrode conductive layer
- 7: leakage preventing agent
- 8: transparent conductive support
- 9: counter electrode

## Claims

1. A dye-sensitized solar cell comprising the following items (a) to (e):
(a) a conductive support;
(b) a porous semiconductor layer;
(c) two kinds of sensitizing dyes that are adsorbed to the porous semiconductor layer and have different molecular sizes, at least one of which has at least one alkyl side chain in the molecule;
(d) a carrier transport layer; and
(e) a counter electrode.

2. The dye-sensitized solar cell according to claim 1, wherein at least one of the two kinds of sensitizing dyes has a photovoltaic characteristic, open circuit voltage of 0.65 V or higher that is exhibited by the dye alone.

3. A dye-sensitized solar cell comprising the following items (a) to (e):
(a) a conductive support;
(b) a porous semiconductor layer;
(c) two kinds of sensitizing dyes that are adsorbed to the porous semiconductor layer and have different molecular sizes, at least one of which has a photovoltaic characteristic, open circuit voltage of 0.65 V or more that is exhibited by the dye alone;
(d) a carrier transport layer; and
(e) a counter electrode.

4. The dye-sensitized solar cell according to claim 3, wherein at least one of the two kinds of sensitizing dyes has at least one alkyl side chain in the molecule.

5. The dye-sensitized solar cell according to any one of claims 1 to 4, wherein the sensitizing dye having a smaller molecular size between the two kinds of sensitizing dyes is an organic dye represented by the following formula (1): 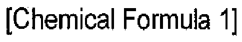 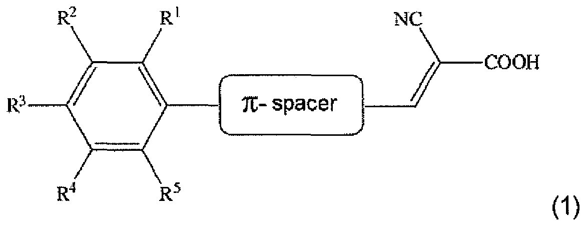 wherein R¹ to R⁶ each independently represent a hydrogen atom, an alkyl group having 1 to 18 carbon atoms, an alkoxy group, a dialkylamino group, or an alicyclic amino group; and the π-spacer represents a divalent aromatic heterocyclic group which may be substituted.

6. The dye-sensitized solar cell according to claim 5, wherein at least one of R¹ to R⁵ represents an alkoxy group, a dialkylamino group or an alicyclic amino group, each having an alkyl side chain moiety having 3 to 16 carbon atoms.

7. The dye-sensitized solar cell according to claim 6, wherein the alkyl side chain moiety is selected from the group consisting of a methyl group, an ethyl group, a propyl group, a butyl group, a pentyl group, a hexyl group, a heptyl group, an octyl group, a hexadecyl group, an isopropyl group, an isobutyl group, an isopentyl group, an isohexyl group, an isoheptyl group, an isooctyl group, a neopentyl group, a neohexyl group, a neoheptyl group, a neooctyl group, a sec-butyl group, a sec-pentyl group, a sec-hexyl group, a sec-heptyl group, a sec-octyl group, a tert-butyl group, a tert-pentyl group, a tert-hexyl group, a tert-heptyl group, a tert-octyl group, a 2-ethylhexyl group, and a 1,1,3,3-tetramethylbutyl group.

8. The dye-sensitized solar cell according to claim 6 or 7, wherein the alicyclic amino group is pyrrolidine or piperidine.

9. The dye-sensitized solar cell according to any one of claims 5 to 8, wherein the aromatic heterocyclic group has any one structure of the following formula (2):

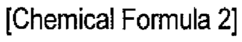

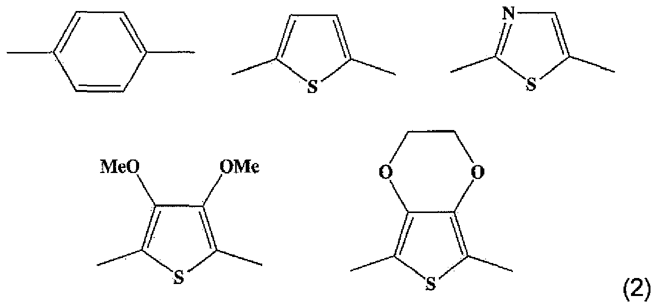

10. The dye-sensitized solar cell according to any one of claims 1 to 9, wherein one of the two kinds of sensitizing dyes is a ruthenium-pyridine complex.

11. The dye-sensitized solar cell according to any one of claims 1 to 9, wherein one of the two kinds of sensitizing dyes is a terpyridine-based ruthenium complex.

12. A sensitizing dye represented by the following formula: 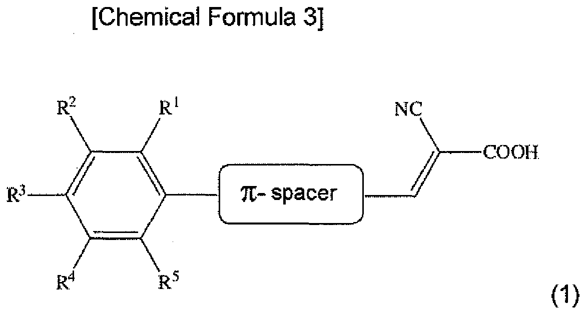 wherein R¹ to R⁵ each independently represent a hydrogen atom, an alkyl group having 1 to 18 carbon atoms, an alkoxy group, a dialkylamino group, or an alicyclic amino group; and the π-spacer represents a divalent aromatic heterocyclic group which may be substituted.

13. The sensitizing dye according to claim 12, wherein at least one of R¹ to R⁵ represents an alkoxy group, a dialkylamino group or an alicyclic amino group, each having an alkyl side chain moiety having 3 to 16 carbon atoms.

14. The sensitizing dye according to claim 13, wherein the alkyl side chain moiety is selected from the group consisting of a methyl group, an ethyl group, a propyl group, a butyl group, a pentyl group, a hexyl group, a heptyl group, an octyl group, a hexadecyl group, an isopropyl group, an isobutyl group, an isopentyl group, an isohexyl group, an isoheptyl group, an isooctyl group, a neopentyl group, a neohexyl group, a neoheptyl group, a neooctyl group, a sec-butyl group, a sec-pentyl group, a sec-hexyl group, a sec-heptyl group, a sec-octyl group, a tert-butyl group, a tert-pentyl group, a tert-hexyl group, a tert-heptyl group, a tert-octyl group, a 2-ethylhexyl group, and a 1,1,3,3-tetramethylbutyl group.

15. The sensitizing dye according to claim 13 or 14, wherein the alicyclic amino group is pyrrolidine or piperidine.

16. The sensitizing dye according to any one of claims 12 to 15, wherein the aromatic heterocyclic group has any one structure represented by the following formula (4):
